# EUROPÄISCHE PATENTSCHRIFT

(11) **EP 1 308 433 B1**
(45) Veröffentlichungstag und Bekanntmachung des Hinweises auf die Patenterteilung: **28.04.2004**
(21) Anmeldenummer: 02023344.1
(22) Anmeldetag: 18.10.2002
(51) Int. Cl.: C07C 69/14, C07C 39/42, C07C 323/19, A01N 31/14, A01N 31/16, A01N 33/10, A01N 37/38, A01N 37/40

(54) **Benzhydrylderivate**
Benzhydryl derivatives
Dérivés de benzydryl

(30) Priorität: 31.10.2001 DE 10153300
(43) Veröffentlichungstag der Anmeldung: 07.05.2003
(73) Patentinhaber: BASF AKTIENGESELLSCHAFT, 67056 Ludwigshafen (DE)
(72) Erfinder: Rose, Ingo, Dr., 68159 Mannheim (DE); Tormo i Blasco, Jordi, Dr., 67117 Limburgerhof (DE); Gewehr, Markus, Dr., 56288 Kastellaun (DE); Grammenos, Wassilios, Dr., 67071 Ludwigshafen (DE); Müller, Bernd, Dr., 67227 Frankenthal (DE); Rheinheimer, Joachim, Dr., 67063 Ludwigshafen (DE); Schäfer, Peter, Dr., 67308 Ottersheim (DE); Schieweck, Frank, Dr., 67258 Hessheim (DE); Grote, Thomas, Dr., 67157 Wachenheim (DE); Gypser, Andreas, Dr., 68159 Mannheim (DE); Ammermann, Eberhard, Dr., 64646 Heppenheim (DE); Lorenz, Gisela, Dr., 67434 Hambach (DE); Stierl, Reinhard, Dr., 67112 Mutterstadt (DE); Strathmann, Siegfried, Dr., 67117 Limburgerhof (DE); Carter, Paul, Dr., 55578 Wolfsheim (DE); Curtze, Jürgen, Dr., 65366 Geisenheim (DE)

(56) Entgegenhaltungen:
- EP-A- 0 015 756
- EP-A- 0 727 141
- US-A- 3 340 294

## Beschreibung

Die Erfindung betrifft Benzhydrylderivate der Formel I, 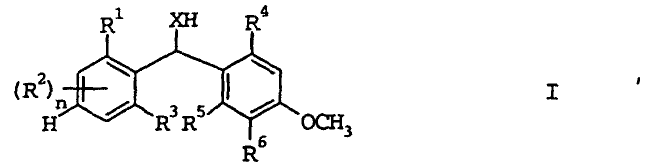 wobei der Index und die Variablen die folgende Bedeutung haben:
- X: Sauerstoff oder Schwefel;
- R¹,R³: Halogen, Cyano, Nitro, Hydroxy, Mercapto, Amino, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkylcarbonyloxy, Formyloxy, C₁-C₆-Alkylthio, C₂-C₆-Alkenylthio, C₂-C₆-Alkinylthio, C₁-C₆-Alkylamino, Di-C₁-C₆-alkylamino, C₁-C₆-Alkylcarbonyl oder Formyl, wobei die Kohlenstoffatome in den genannten Resten partiell oder vollständig halogeniert sein können;
- R²: Halogen, Cyano, Nitro, Hydroxy, Mercapto, Amino, C₁-C₆-Alkyl, C₁-C₆-Alkoxy, C₁-C₆-Haloalkyl oder C₁-C₆-Haloalkoxy, wobei die Gruppen R² verschieden sein können, wenn n=2 ist;
- R⁴: C₁-C₆-Alkyl, C₂-C₆-Alkenyl oder C₂-C₆-Alkinyl, wobei die Kohlenstoffatome in diesen Resten unsubstituiert oder teilweise oder vollständig halogeniert sein können;
- R⁵,R⁶: Hydroxy, C₁-C₆-Alkyl, C₂-C₆-Alkenyl, C₁-C₆-Haloalkyl, C₂-C₆-Haloalkenyl, C₁-C₆-Alkoxy, C₂-C₆-Alkenyloxy, C₁-C₆-Haloalkoxy, C₂-C₆-Haloalkenyloxy, C₃-C₈-Cycloalkyl, C₃-C₈-Cycloalkyl-C₁-C₃-alkyl, C₃-C₈-Cycloalkoxy oder C₃-C₈-Cycloalkyl-C₁-C₃-alkoxy;
- n: 0, 1 oder 2;

Zusätzlich betrifft die Erfindung Verfahren zur Herstellung der Verbindungen I, sie enthaltende Mittel und die Verwendung der Verbindungen I zur Bekämpfung von pflanzenpathogenen Schadpilzen.

Verschiedene Benzhydrylalkohole als Edukte für Pharmazeutika sind in JP 08225474 beschrieben.

Aus EP-A 461 079 sind verschiedene Benzhydrylalkohole mit herbizider Wirkung bekannt.

Benzhydrylalkohole mit fungizider Wirkung sind bereits bekannt, beispielsweise (RS)-2,4'-difluoro-alpha-(1H-1,2,4-triazol-1-ylmethyl)benzhydrylalkohol (Flutriafol; EP-A 015 756) oder (R,S)-2,4'-dichloro-alpha-(pyrimidin-5-yl)benzhydrylalkohol (Fenarimol; GB 1 218 623). Ihre Wirkung konnte jedoch nicht in jeder Hinsicht befriedigen.

Benzophenone mit fungizider Wirkung sind aus EP-A 727 141 bekannt.

Es ist Aufgabe dieser Erfindung, Benzhydrylderivate mit verbesserter Wirksamkeit in der Bekämpfung von Schadpilzen bereitzustellen.

Demgemäß wurden die Benzhydrylderivate der Formel I gefunden. Weiterhin wurden Verfahren zur Herstellung der Verbindungen I und diese enthaltende Mittel zur Bekämpfung von Schadpilzen gefunden.

Die Verbindungen der Formel I unterscheiden sich von den in JP 08225474 offenbarten Benzhydrylalkoholen durch das Substitutionsmuster an den Phenylringen.

Die Verbindungen der Formel I unterscheiden sich von den in EP-A 461 079 offenbarten herbizid wirksamen Benzhydrylalkoholen durch die in 2,2,2',2',3',4 und 4'-Position fixierten Substituenten der Phenylringe.

Verbindungen der Formel I sind sekundäre Alkohole bzw. Thiole, während es sich bei den in EP-A 015 756 und GB 1 218 623 offenbarten Verbindungen um tertiäre Alkohole handelt, die sich außerdem durch das Substitutionsmuster an den Phenylringen von Verbindungen der Formel I unterscheiden.

Die Verbindungen der Formel I unterscheiden sich von den in EP-A 727 141 offenbarten fungizid wirksamen Benzophenonen durch den Ersatz der Ketogruppe durch eine Alkohol- bzw. Thiolfunktion sowie durch die Substitutionsmuster an den Phenylringen.
Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom oder Jod, bevorzugt Chlor oder Brom;
**Alkyl:** gesättigte, geradkettige oder verzweigte Kohlenwasserstoffreste mit 1 bis 4 oder 1 bis 6, Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, , 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 6 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 6 Kohlenstoffatomen (wie vorstehend genannt), welche über ein Sauerstoffatom (-O-) an den Phenylring gebunden sind;
**Halogenalkoxy:** geradkettige oder verzweigte Halogenalkylgruppen mit 1 bis 6 Kohlenstoffatomen (wie vorstehend genannt), welche über ein Sauerstoffatom (-O-) an den Phenylring gebunden sind;
**Alkylthio:** geradkettige oder verzweigte Alkylgruppen mit 1 bis 6 oder 1 bis 4 Kohlenstoffatomen (wie vorstehend genannt), welche über ein Schwefelatom (-S-) an den Phenylring gebunden sind;
**Alkylamino:** eine geradkettige oder verzweigte Alkylgruppe mit 1 bis 6 Kohlenstoffatomen (wie vorstehend genannt), welche über eine Aminogruppe (-NH-) an den Phenylring gebunden ist;
**Dialkylamino:** zwei voneinander unabhängige geradkettige oder verzweigte Alkylgruppen mit jeweils 1 bis 6 Kohlenstoffatomen (wie vorstehend genannt), welche über ein Stickstoffatom an den Phenylring gebunden sind;
**Alkylcarbonyloxy:** eine geradkettige oder verzweigte Alkylgruppe mit 1 bis 6 Kohlenstoffatomen (wie vorstehend genannt), welche über eine Carbonyloxygruppe (-CO₂-) an den Phenylring gebunden ist;
**Alkenyl:** ungesättigte, geradkettige oder verzweigte Kohlenwasserstoffreste mit 2 bis 6 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und 1-Ethyl-2-methyl-2-propenyl;
**Halogenalkenyl:** ungesättigte, geradkettige oder verzweigte Kohlenwasserstoffreste mit 2 bis 6 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkenyloxy:** ungesättigte, geradkettige oder verzweigte Kohlenwasserstoffreste mit 2 bis 6 Kohlenstoffatomen und einer Doppelbindung in einer beliebigen, nicht zum Heteroatom benachbarten, Position (wie vorstehend genannt), welche über ein Sauerstoffatom (-O-) an den Phenylring gebunden sind;
**Halogenalkenyloxy:** ungesättigte, geradkettige oder verzweigte Alkenyloxygruppen mit 3 bis 6 Kohlenstoffatomen (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkenylthio:** ungesättigte, geradkettige oder verzweigte Kohlenwasserstoffreste mit 2 bis 6 Kohlenstoffatomen und einer Doppelbindung in einer beliebigen, nicht zum Heteroatom benachbarten, Position (wie vorstehend genannt), welche über ein Schwefelatom (-S-) an den Phenylring gebunden sind;
**Alkinyl:** geradkettige oder verzweigte Kohlenwasserstoffgruppen mit 2 bis 6 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkinyloxy:** ungesättigte, geradkettige oder verzweigte Kohlenwasserstoffreste mit 3 bis 6 Kohlenstoffatomen und einer Dreifachbindung in einer beliebigen, nicht zum Heteroatom benachbarten, Position (wie vorstehend genannt), welche über ein Sauerstoffatom (-O-) an das Gerüst gebunden sind;
**Alkinylthio:** ungesättigte, geradkettige oder verzweigte Kohlenwasserstoffreste mit 3 bis 6 Kohlenstoffatomen und einer Dreifachbindung in einer beliebigen, nicht zum Heteroatom benachbarten, Position (wie vorstehend genannt), welche über ein Schwefelatom (-S-) an das Gerüst gebunden sind;

Im Hinblick auf ihre bestimmungsgemäße Verwendung der Benzhydrylverbindungen der Formel I sind die folgenden Bedeutungen der Substituenten, und zwar jeweils für sich allein oder in Kombination, besonders bevorzugt:

Benzhydrylderivate der Formel I gemäß Anspruch 1, wobei die Variablen die folgende Bedeutung haben:
- X: Sauerstoff oder Schwefel;
- R¹,R³: unabhängig voneinander Halogen, Hydroxy, Mercapto, Amino, C₁-C₆-Alkyl, C₂-C₆-Alkenyl, C₂-C₆-Alkinyl, C₁-C₆-Haloalkyl, C₂-C₆-Haloalkenyl, C₂-C₆-Haloalkinyl, C₁-C₆-Alkoxy, C₂-C₆-Alkenyloxy, C₂-C₆-Alkinyloxy, C₁-C₆-Haloalkoxy, C₂-C₆-Haloalkenyloxy, C₂-C₆-Haloalkinyloxy, C₁-C₆-Alkylcarbonyloxy, C₁-C₆-Haloalkylcarbonyloxy, Formyloxy, C₁-C₆-Alkylthio, C₂-C₆-Alkenylthio, C₂-C₆-Alkinylthio, C₁-C₆-Haloalkylthio, C₂-C₆-Haloalkenylthio, C₂-C₆-Haloalkinylthio, C₁-C₆-Alkylamino, Di- C₁-C₆-alkylamino, C₁-C₆-Haloalkylamino, Di-C₁-C₆-haloalkylamino, C₁-C₆-Alkylcarbonyl, C₁-C₆-Haloalkylcarbonyl oder Formyl;
- R²: Halogen, Hydroxy, C₁-C₆-Alkyl, C₁-C₆-Alkoxy, C₁-C₆-Haloalkyl oder C₁-C₆-Haloalkoxy, wobei die Reste R² verschieden sein können, wenn n=2 ist.
- R⁴: C₁-C₆-Alkyl, C₂-C₆-Alkenyl, C₂-C₆-Alkinyl, C₁-C₆-Haloalkyl, C₂-C₆-Haloalkenyl oder C₂-C₆-Haloalkinyl;
- R⁵,R⁶: unabhängig voneinander Hydroxy, C₁-C₆-Alkoxy, C₂-C₆-Alkenyloxy, C₁-C₆-Haloalkoxy oder C₂-C₆-Haloalkenyloxy;
- n: 0, 1 oder 2.

Besonders bevorzugt werden Verbindungen I, in denen X Sauerstoff oder Schwefel ist;
R¹ und R³ unabhängig voneinander Halogen, Hydroxy, Mercapto, Amino, C₁-C₆-Alkyl, C₁-C₆-Haloalkyl, C₁-C₆-Alkoxy, C₁-C₆-Haloalkoxy oder C₁-C₆-Alkylcarbonyl sind;
R² Halogen, Hydroxy, C₁-C₆-Alkyl, C₁-C₆-Haloalkyl, C₁-C₆-Alkoxy, oder C₁-C₆-Haloalkoxy ist, wobei die Reste R² verschieden sein können, wenn n=2 ist;
R⁴ Methyl ist;
R⁵,R⁶ unabhängig voneinander Hydroxy, C₁-C₆-Alkoxy, C₂-C₆-Alkenyloxy, C₁-C₆-Haloalkoxy oder C₂-C₆-Haloalkenyloxy sind, und
n 0 oder 1 ist.

Insbesondere werden Verbindungen I bevorzugt, in denen X für Sauerstoff steht.

Außerdem werden Verbindungen I bevorzugt, in denen R¹ und R³ unabhängig voneinander Halogen, Hydroxy, Amino, Mercapto, Nitro, Cyano, C₁-C₆-Alkyl, C₁-C₆-Haloalkyl, C₁-C₆-Alkoxy, C₁-C₆-Haloalkoxy, C₁-C₆-Alkylcarbonyloxy, C₁-C₆-Haloalkylcarbonyloxy, C₁-C₆-Alkylthio, C₁-C₆-Haloalkylthio, C₁-C₆-Alkylcarbonyl oder C₁-C₆-Haloalkylcarbonyl bedeuten. Insbesondere sind hier zu nennen: Fluor, Chlor, Brom oder Iod, Hydroxy, Mercapto, Amino, Methyl, Ethyl, Methoxy, Ethoxy, Methylcarbonyloxy, Ethylcarbonyloxy, Methylthio, Ethylthio, Trifluormethyl, Difluormethoxy oder Chlorfluormethoxy.

Daneben werden Verbindungen I bevorzugt, in denen R¹ und R³ unabhängig voneinander Halogen, Hydroxy, Mercapto, Amino, C₁-C₆-Alkyl, C₁-C₆-Haloalkyl, C₁-C₆-Alkoxy oder C₁-C₆-Haloalkoxy bedeuten. Insbesondere sind hier zu nennen: Fluor, Chlor, Brom oder Iod, Hydroxy, Mercapto, Amino, Methyl, Ethyl, Methoxy, Ethoxy, Trifluormethyl, Difluormethoxy oder Chlorfluormethoxy.

Ferner werden Verbindungen I besonders bevorzugt, in denen R¹ und R³ unabhängig voneinander für Halogen wie Fluor, Chlor, Brom oder Iod, Hydroxy, C₁-C₆-Alkyl wie Methyl oder Ethyl, C₁-C₆-Alkoxy wie Methoxy oder Ethoxy oder C₁-C₆-Alkylcarbonyloxy wie Methylcarbonyloxy oder Ethylcarbonyloxy stehen.

Daneben werden Verbindungen I besonders bevorzugt, in denen R¹ und R³ unabhängig voneinander für Halogen wie Fluor, Chlor, Brom oder Iod, Hydroxy, C₁-C₆-Alkyl wie Methyl oder Ethyl oder C₁-C₆-Alkoxy wie Methoxy oder Ethoxy stehen.

Ganz besonders bevorzugt sind Verbindungen I, in denen R¹ und R³ unabhängig voneinander für Halogen wie Chlor oder Brom oder C₁-C₆-Alkyl wie Methyl oder Ethyl stehen.

Gleichermaßen ganz besonders bevorzugt werden Verbindungen I, in denen R¹ und R³ unabhängig voneinander für Hydroxy, C₁-C₆-Alkoxy wie Methoxy oder Ethoxy oder C₁-C₆-Alkylcarbonyloxy wie Methylcarbonyloxy oder Ethylcarbonyloxy stehen.

Daneben werden Verbindungen I ganz besonders bevorzugt, in denen R¹ und R³ unabhängig voneinander für Hydroxy oder C₁-C₆-Alkoxy wie Methoxy oder Ethoxy stehen.

Außerdem werden auch Verbindungen I bevorzugt, in denen R² für Halogen, Hydroxy, C₁-C₆-Alkyl, C₁-C₆-Haloalkyl, C₁-C₆-Alkoxy oder C₁-C₆-Haloalkoxy steht, wobei die Gruppen R² verschieden sein können, wenn n=2 ist. Insbesondere sind hier zu nennen: Fluor, Chlor, Brom, Iod, Methyl, Ethyl, Trifluormethyl, Difluormethoxy, Chlorfluormethoxy.

Insbesondere werden auch Verbindungen I bevorzugt, in denen R² für Halogen, Hydroxy oder C₁-C₆-Alkoxy wie Methoxy oder Ethoxy steht.

Verbindungen I werden ganz besonders bevorzugt, in denen R² für Halogen, insbesondere Brom oder Chlor, steht.

Außerdem werden Verbindungen I besonders bevorzugt, in denen R⁴, C₁-C₆-Alkyl oder C₁-C₆-Haloalkyl bedeutet. Insbesondere sind hier zu nennen: Methyl, Ethyl und Trifluormethyl.

Insbesondere werden Verbindungen I bevorzugt, in denen R⁴ für Methyl steht.

Außerdem besonders bevorzugt werden Verbindungen I, in denen R⁵ und R⁶ unabhängig voneinander Hydroxy, C₁-C₆-Alkoxy, C₂-C₆-Alkenyloxy, C₁-C₆-Haloalkoxy oder C₂-C₆-Haloalkenyloxy bedeuten. Insbesondere sind hier zu nennen: Hydroxy, Methoxy, Ethoxy, n-Propoxy, i-Propoxy, Butoxy, Pentoxy, Hexoxy, Propenyloxy, Trifluormethoxy, Difluormethoxy und Chlorfluormethoxy.

Besonders bevorzugt sind ferner Verbindungen I, in denen R⁵ und R⁶ unabhängig voneinander für Hydroxy oder C₁-C₆-Alkoxy wie Methoxy, Ethoxy, Propoxy, Butoxy, Pentoxy und Hexoxy stehen.

Ganz besonders bevorzugt sind Verbindungen I, in denen R⁵ und R⁶ Methoxy bedeuten.

Daneben werden Verbindungen I besonders bevorzugt, in denen n 0 oder 1, ganz besonders bevorzugt 1 ist.

Ganz besonders bevorzugt werden Verbindungen I, in denen R¹ und R³ unabhängig voneinander Fluor, Chlor, Brom, Iod, Hydroxy, Mercapto, Methylthio, Amino, Methyl, Ethyl, Trifluormethyl, Methoxy, Difluormethoxy, Fluorchlormethoxy, Trifluormethoxy, Methylcarbonyloxy oder Ethylcarbonyloxy bedeuten, R² Fluor, Chlor, Brom, Iod, Hydroxy, Methyl, Trifluormethyl, Methoxy, Ethoxy, Difluormethoxy oder Chlorfluormethoxy bedeutet, R⁴ Methyl, Ethyl oder Trifluormethyl bedeutet, R⁵ und R⁶ unabhängig voneinander Hydroxy, Methoxy, Ethoxy, n-Propoxy, i-Propoxy oder n-Butoxy bedeuten und n 0 oder 1 bedeutet.

Insbesondere sind im Hinblick auf ihre Verwendung die in den folgenden Tabellen zusammengestellten Verbindungen I-A bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 1

Verbindungen der allgemeinen Formel I-A, in denen n 0 ist, R⁴ für Methyl und R⁶ für Methoxy steht und die Kombination der Reste R¹, R³, R⁵ und für eine Verbindung jeweils einer Zeile der Tabelle A entspricht

### Tabelle 2

Verbindungen der allgemeinen Formel I-A, in denen n für 1, R² für 3-Methyl, R⁴ für Methyl und R⁶ für Methoxy steht und die Kombination der Reste R¹, R³ und R⁵ für eine Verbindung jeweils einer Zeile der Tabelle A entspricht

### Tabelle 3

Verbindungen der allgemeinen Formel I-A, in denen n für 1, R² für 5-Methyl, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 4

Verbindungen der allgemeinen Formel I-A, in denen n für 1, R² für 3-Trifluormethyl, R⁴ für Methyl und R⁶ für Methoxy steht und die Kombination der Reste R¹, R³ und R⁵ für eine Verbindung jeweils einer Zeile der Tabelle A entspricht

### Tabelle 5

Verbindungen der allgemeinen Formel I-A, in denen n für 1, R² für 5-Trifluormethyl, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 6

Verbindungen der allgemeinen Formel I-A, in denen n für 1, R² für 3-Hydroxy, R⁴ für Methyl und R⁶ für Methoxy steht und die Kombination der Reste R¹, R³ und R⁵ für eine Verbindung jeweils einer Zeile der Tabelle A entspricht

### Tabelle 7

Verbindungen der allgemeinen Formel I-A, in denen n für 1, R² für 5-Hydroxy, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 8

Verbindungen der allgemeinen Formel I-A, in denen n für 1, R² für 3-Methoxy, R⁴ für Methyl und R⁶ für Methoxy steht und die Kombination der Reste R¹, R³ und R⁵ für eine Verbindung jeweils einer Zeile der Tabelle A entspricht

### Tabelle 9

Verbindungen der allgemeinen Formel I-A, in denen n für 1, R² für 5-Methoxy, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 10

Verbindungen der allgemeinen Formel I-A, in denen n für 1, R² für 3-Ethoxy, R⁴ für Methyl und R⁶ für Methoxy steht und die Kombination der Reste R¹, R³ und R⁵ für eine Verbindung jeweils einer Zeile der Tabelle A entspricht

### Tabelle 11

Verbindungen der allgemeinen Formel I-A, in denen n für 1, R² für 5-Ethoxy, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 12

Verbindungen der allgemeinen Formel I-A, in denen n für 1, R² für 3-Difluormethoxy, R⁴ für Methyl und R⁶ für Methoxy steht und die Kombination der Reste R¹, R³ und R⁵ für eine Verbindung jeweils einer Zeile der Tabelle A entspricht

### Tabelle 13

Verbindungen der allgemeinen Formel I-A, in denen n für 1, R² für 5-Difluormethoxy, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 14

Verbindungen der allgemeinen Formel I-A, in denen n für 1, R² für 3-Chlorfluormethoxy, R⁴ für Methyl und R⁶ für Methoxy steht und die Kombination der Reste R¹, R³ und R⁵ für eine Verbindung jeweils einer Zeile der Tabelle A entspricht

### Tabelle 15

Verbindungen der allgemeinen Formel I-A, in denen n für 1, R² für 5-Chlorfluormethoxy, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 16

Verbindungen der allgemeinen Formel I-A, in denen n für 1, R² für 3-Fluor, R⁴ für Methyl und R⁶ für Methoxy steht und die Kombination der Reste R¹, R³ und R⁵ für eine Verbindung jeweils einer Zeile der Tabelle A entspricht

### Tabelle 17

Verbindungen der allgemeinen Formel I-A, in denen n für 1, R² für 5-Fluor, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 18

Verbindungen der allgemeinen Formel I-A, in denen n für 1, R² für 3-Chlor, R⁴ für Methyl und R⁶ für Methoxy steht und die Kombination der Reste R¹, R³ und R⁵ für eine Verbindung jeweils einer Zeile der Tabelle A entspricht

### Tabelle 19

Verbindungen der allgemeinen Formel I-A, in denen n für 1, R² für 5-Chlor, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 20

Verbindungen der allgemeinen Formel I-A, in denen n für 1, R² für 3-Brom, R⁴ für Methyl und R⁶ für Methoxy steht und die Kombination der Reste R¹, R³ und R⁵ für eine Verbindung jeweils einer Zeile der Tabelle A entspricht

### Tabelle 21

Verbindungen der allgemeinen Formel I-A, in denen n für 1, R² für 5-Brom, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 22

Verbindungen der allgemeinen Formel I-A, in denen n für 1, R² für 3-Iod, R⁴ für Methyl und R⁶ für Methoxy steht und die Kombination der Reste R¹, R³ und R⁵ für eine Verbindung jeweils einer Zeile der Tabelle A entspricht

### Tabelle 23

Verbindungen der allgemeinen Formel I-A, in denen n für 1, R² für 5-Iod, R⁴ für Methyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 24

Verbindungen der allgemeinen Formel I-A, in denen n 0 ist, R⁴ für Ethyl und R⁶ für Methoxy steht und die Kombination der Reste R¹, R³, R⁵ und für eine Verbindung jeweils einer Zeile der Tabelle A entspricht

### Tabelle 25

Verbindungen der allgemeinen Formel I-A, in denen n für 1, R² für 3-Methyl, R⁴ für Ethyl und R⁶ für Methoxy steht und die Kombination der Reste R¹, R³, und R⁵ für eine Verbindung jeweils einer Zeile der Tabelle A entspricht

### Tabelle 26

Verbindungen der allgemeinen Formel I-A, in denen n für 1, R² für 5-Methyl, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 27

Verbindungen der allgemeinen Formel I-A, in denen n für 1, R² für 3-Trifluormethyl, R⁴ für Ethyl und R⁶ für Methoxy steht und die Kombination der Reste R¹, R³ und R⁵ für eine Verbindung jeweils einer Zeile der Tabelle A entspricht

### Tabelle 28

Verbindungen der allgemeinen Formel I-A, in denen n für 1, R² für 5-Trifluormethyl, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 29

Verbindungen der allgemeinen Formel I-A, in denen n für 1, R² für 3-Hydroxy, R⁴ für Ethyl und R⁶ für Methoxy steht und die Kombination der Reste R¹, R³ und R⁵ für eine Verbindung jeweils einer Zeile der Tabelle A entspricht

### Tabelle 30

Verbindungen der allgemeinen Formel I-A, in denen n für 1, R² für 5-Hydroxy, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 31

Verbindungen der allgemeinen Formel I-A, in denen n für 1, R² für 3-Hydroxy, R⁴ für Ethyl und R⁶ für Methoxy steht und die Kombination der Reste R¹, R³ und R⁵ für eine Verbindung jeweils einer Zeile der Tabelle A entspricht

### Tabelle 32

Verbindungen der allgemeinen Formel I-A, in denen n für 1, R² für 5-Methoxy, R⁴ für Ethyl und R⁶ für Methoxy steht und die Kombination der Reste R¹, R³ und R⁵ für eine Verbindung jeweils einer , Zeile der Tabelle A entspricht, wobei R¹ ungleich R³ ist

### Tabelle 33

Verbindungen der allgemeinen Formel I-A, in denen n für 1, R² für 3-Ethoxy, R⁴ für Ethyl und R⁶ für Methoxy steht und die Kombination der Reste R¹, R³ und R⁵ für eine Verbindung jeweils einer Zeile der Tabelle A entspricht

### Tabelle 34

Verbindungen der allgemeinen Formel I-A, in denen n für 1, R² für 5-Ethoxy, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 35

Verbindungen der allgemeinen Formel I-A, in denen n für 1, R² für 3-Difluormethoxy, R⁴ für Ethyl und R⁶ für Methoxy steht und die Kombination der Reste R¹, R³ und R⁵ für eine Verbindung jeweils einer Zeile der Tabelle A entspricht

### Tabelle 36

Verbindungen der allgemeinen Formel I-A, in denen n für 1, R² für 5-Difluormethoxy, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 37

Verbindungen der allgemeinen Formel I-A, in denen n für 1, R² für 3-Chlorfluormethoxy, R⁴ für Ethyl und R⁶ für Methoxy steht und die Kombination der Reste R¹, R³ und R⁵ für eine Verbindung jeweils einer Zeile der Tabelle A entspricht

### Tabelle 38

Verbindungen der allgemeinen Formel I-A, in denen n für 1, R² für 5-Chlorfluormethoxy, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 39

Verbindungen der allgemeinen Formel I-A, in denen n für 1, R² für 3-Fluor, R⁴ für Ethyl und R⁶ für Methoxy steht und die Kombination der Reste R¹, R³ und R⁵ für eine Verbindung jeweils einer Zeile der Tabelle A entspricht

### Tabelle 40

Verbindungen der allgemeinen Formel I-A, in denen n für 1, R² für 5-Fluor, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 41

Verbindungen der allgemeinen Formel I-A, in denen n für 1, R² für 3-Chlor, R⁴ für Ethyl und R⁶ für Methoxy steht und die Kombination der Reste R¹, R³ und R⁵ für eine Verbindung jeweils einer Zeile der Tabelle A entspricht

### Tabelle 42

Verbindungen der allgemeinen Formel I-A, in denen n für 1, R² für 5-Chlor, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 43

Verbindungen der allgemeinen Formel I-A, in denen n für 1, R² für 3-Brom, R⁴ für Ethyl und R⁶ für Methoxy steht und die Kombination der Reste R¹, R³ und R⁵ für eine Verbindung jeweils einer Zeile der Tabelle A entspricht

### Tabelle 44

Verbindungen der allgemeinen Formel I-A, in denen n für 1, R² für 5-Brom, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 45

Verbindungen der allgemeinen Formel I-A, in denen n für 1, R² für 3-Iod, R⁴ für Ethyl und R⁶ für Methoxy steht und die Kombination der Reste R¹, R³ und R⁵ für eine Verbindung jeweils einer Zeile der Tabelle A entspricht

### Tabelle 46

Verbindungen der allgemeinen Formel I-A, in denen n für 1, R² für 5-Iod, R⁴ für Ethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 47

Verbindungen der allgemeinen Formel I-A, in denen n 0 ist, R⁴ für Trifluormethyl und R⁶ für Methoxy steht und die Kombination der Reste R¹, R³, R⁵ und für eine Verbindung jeweils einer Zeile der Tabelle A entspricht

### Tabelle 48

Verbindungen der allgemeinen Formel I-A, in denen n für 1, R² für 3-Methyl, R⁴ für Trifluormethyl und R⁶ für Methoxy steht und die Kombination der Reste R¹, R³ und R⁵ für eine Verbindung jeweils einer Zeile der Tabelle A entspricht

### Tabelle 49

Verbindungen der allgemeinen Formel I-A, in denen n für 1, R² für 5-Methyl, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 50

Verbindungen der allgemeinen Formel I-A, in denen n für 1, R² für 3-Trifluormethyl, R⁴ für Trifluormethyl und R⁶ für Methoxy steht und die Kombination der Reste R¹, R³ und R⁵ für eine Verbindung jeweils einer Zeile der Tabelle A entspricht

### Tabelle 51

Verbindungen der allgemeinen Formel I-A, in denen n für 1, R² für 5-Trifluormethyl, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 52

Verbindungen der allgemeinen Formel I-A, in denen n für 1, R² für 3-Hydroxy, R⁴ für Trifluormethyl und R⁶ für Methoxy steht und die Kombination der Reste R¹, R³ und R⁵ für eine Verbindung jeweils einer Zeile der Tabelle A entspricht

### Tabelle 53

Verbindungen der allgemeinen Formel I-A, in denen n für 1, R² für 5-Hydroxy, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 54

Verbindungen der allgemeinen Formel I-A, in denen n für 1, R² für 3-Methoxy, R⁴ für Trifluormethyl und R⁶ für Methoxy steht und die Kombination der Reste R¹, R³ und R⁵ für eine Verbindung jeweils einer Zeile der Tabelle A entspricht

### Tabelle 55

Verbindungen der allgemeinen Formel I-A, in denen n für 1, R² für 5-Methoxy, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist ,

### Tabelle 56

Verbindungen der allgemeinen Formel I-A, in denen n für 1, R² für 3-Ethoxy, R⁴ für Trifluormethyl und R⁶ für Methoxy steht und die Kombination der Reste R¹, R³ und R⁵ für eine Verbindung jeweils einer Zeile der Tabelle A entspricht

### Tabelle 57

Verbindungen der allgemeinen Formel I-A, in denen n für 1, R² für 5-Ethoxy, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 58

Verbindungen der allgemeinen Formel I-A, in denen n für 1, R² für 3-Difluormethoxy, R⁴ für Trifluormethyl und R⁶ für Methoxy steht und die Kombination der Reste R¹, R³ und R⁵ für eine Verbindung jeweils einer Zeile der Tabelle A entspricht

### Tabelle 59

Verbindungen der allgemeinen Formel I-A, in denen n für 1, R² für 5-Difluormethoxy, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 60

Verbindungen der allgemeinen Formel I-A, in denen n für 1, R² für 3-Chlorfluormethoxy, R⁴ für Trifluormethyl und R⁶ für Methoxy steht und die Kombination der Reste R¹, R³ und R⁵ für eine Verbindung jeweils einer Zeile der Tabelle A entspricht

### Tabelle 61

Verbindungen der allgemeinen Formel I-A, in denen n für 1, R² für 5-Chlorfluormethoxy, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 62

Verbindungen der allgemeinen Formel I-A, in denen n für 1, R² für 3-Fluor, R⁴ für Trifluormethyl und R⁶ für Methoxy steht und die Kombination der Reste R¹, R³ und R⁵ für eine Verbindung jeweils einer Zeile der Tabelle A entspricht

### Tabelle 63

Verbindungen der allgemeinen Formel I-A, in denen n für 1, R² für 5-Fluor, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 64

Verbindungen der allgemeinen Formel I-A, in denen n für 1, R² für 3-Chlor, R⁴ für Trifluormethyl und R⁶ für Methoxy steht und die Kombination der Reste R¹, R³ und R⁵ für eine Verbindung jeweils einer Zeile der Tabelle A entspricht

### Tabelle 65

Verbindungen der allgemeinen Formel I-A, in denen n für 1, R² für 5-Chlor, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 66

Verbindungen der allgemeinen Formel I-A, in denen n für 1, R² für 3-Brom, R⁴ für Trifluormethyl und R⁶ für Methoxy steht und die Kombination der Reste R¹, R³ und R⁵ für eine Verbindung jeweils einer Zeile der Tabelle A entspricht

### Tabelle 67

Verbindungen der allgemeinen Formel I-A, in denen n für 1, R² für 5-Brom, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 68

Verbindungen der allgemeinen Formel I-A, in denen n für 1, R² für 3-Iod, R⁴ für Trifluormethyl und R⁶ für Methoxy steht und die Kombination der Reste R¹, R³ und R⁵ für eine Verbindung jeweils einer Zeile der Tabelle A entspricht

### Tabelle 69

Verbindungen der allgemeinen Formel I-A, in denen n für 1, R² für 5-Iod, R⁴ für Trifluormethyl und R⁶ für Methoxy steht und die Kombination der Reste R¹, R³ und R⁵ für eine Verbindung jeweils einer Zeile der Tabelle A entspricht, wobei R¹ ungleich R³ ist

### Tabelle 70

Verbindungen der allgemeinen Formel I-A, in denen n 0 ist, R⁴ für Methyl und R⁶ für Ethoxy steht und die Kombination der Reste R¹, R³, R⁵ und für eine Verbindung jeweils einer Zeile der Tabelle A entspricht

### Tabelle 71

Verbindungen der allgemeinen Formel I-A, in denen n für 1, R² für 3-Methyl, R⁴ für Methyl und R⁶ für Ethoxy steht und die Kombination der Reste R¹, R³, und R⁵ für eine Verbindung jeweils einer Zeile der Tabelle A entspricht

### Tabelle 72

Verbindungen der allgemeinen Formel I-A, in denen n für 1, R² für 5-Methyl, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 73

Verbindungen der allgemeinen Formel I-A, in denen n für 1, R² für 3-Trifluormethyl, R⁴ für Methyl und R⁶ für Ethoxy steht und die Kombination der Reste R¹, R³ und R⁵ für eine Verbindung jeweils einer Zeile der Tabelle A entspricht

### Tabelle 74

Verbindungen der allgemeinen Formel I-A, in denen n für 1, R² für 5-Trifluormethyl, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 75

Verbindungen der allgemeinen Formel I-A, in denen n für 1, R² für 3-Hydroxy, R⁴ für Methyl und R⁶ für Ethoxy steht und die Kombination der Reste R¹, R³ und R⁵ für eine Verbindung jeweils einer Zeile der Tabelle A entspricht

### Tabelle 76

Verbindungen der allgemeinen Formel I-A, in denen n für 1, R² für 5-Hydroxy, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 77

Verbindungen der allgemeinen Formel I-A, in denen n für 1, R² für 3-Hydroxy, R⁴ für Methyl und R⁶ für Ethoxy steht und die Kombination der Reste R¹, R³ und R⁵ für eine Verbindung jeweils einer Zeile der Tabelle A entspricht

### Tabelle 78

Verbindungen der allgemeinen Formel I-A, in denen n für 1, R² für 5-Methoxy, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 79

Verbindungen der allgemeinen Formel I-A, in denen n für 1, R² für 3-Ethoxy, R⁴ für Methyl und R⁶ für Ethoxy steht und die Kombination der Reste R¹, R³ und R⁵ für eine Verbindung jeweils einer Zeile der Tabelle A entspricht

### Tabelle 80

Verbindungen der allgemeinen Formel I-A, in denen n für 1, R² für 5-Methoxy, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 81

Verbindungen der allgemeinen Formel I-A, in denen n für 1, R² für 3-Difluormethoxy, R⁴ für Methyl und R⁶ für Ethoxy steht und die Kombination der Reste R¹, R³ und R⁵ für eine Verbindung jeweils einer Zeile der Tabelle A entspricht

### Tabelle 82

Verbindungen der allgemeinen Formel I-A, in denen n für 1, R² für 5-Difluormethoxy, R⁴ für Methyl und R⁶ für Ethoxysteht und die Kombination der Reste R¹, R³ und R⁵ für eine Verbindung jeweils einer Zeile der Tabelle A entspricht, wobei R¹ ungleich R³ ist

### Tabelle 83

Verbindungen der allgemeinen Formel I-A, in denen n für 1, R² für 3-Chlorfluormethoxy, R⁴ für Methyl und R⁶ für Ethoxy steht und die Kombination der Reste R¹, R³ und R⁵ für eine Verbindung jeweils einer Zeile der Tabelle A entspricht

### Tabelle 84

Verbindungen der allgemeinen Formel I-A, in denen n für 1, R² für 5-Chlorfluormethoxy, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 85

Verbindungen der allgemeinen Formel I-A, in denen n für 1, R² für 3-Fluor, R⁴ für Methyl und R⁶ für Ethoxy steht und die Kombination der Reste R¹, R³ und R⁵ für eine Verbindung jeweils einer Zeile der Tabelle A entspricht

### Tabelle 86

Verbindungen der allgemeinen Formel I-A, in denen n für 1, R² für 5-Fluor, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 87

Verbindungen der allgemeinen Formel I-A, in denen n für 1, R² für 3-Chlor, R⁴ für Methyl und R⁶ für Ethoxy steht und die Kombination der Reste R¹, R³ und R⁵ für eine Verbindung jeweils einer Zeile der Tabelle A entspricht

### Tabelle 88

Verbindungen der allgemeinen Formel I-A, in denen n für 1, R² für 5-Chlor, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 89

Verbindungen der allgemeinen Formel I-A, in denen n für 1, R² für 3-Brom, R⁴ für Methyl und R⁶ für Ethoxy steht und die Kombination der Reste R¹, R³ und R⁵ für eine Verbindung jeweils einer Zeile der Tabelle A entspricht

### Tabelle 90

Verbindungen der allgemeinen Formel I-A, in denen n für 1, R² für 5-Brom, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 91

Verbindungen der allgemeinen Formel I-A, in denen n für 1, R² für 3-Iod, R⁴ für Methyl und R⁶ für Ethoxy steht und die Kombination der Reste R¹, R³ und R⁵ für eine Verbindung jeweils einer Zeile der Tabelle A entspricht

### Tabelle 92

Verbindungen der allgemeinen Formel I-A, in denen n für 1, R² für 5-Iod, R⁴ für Methyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 93

Verbindungen der allgemeinen Formel I-A, in denen n 0 ist, R⁴ für Ethyl und R⁶ für Ethoxy steht und die Kombination der Reste R¹, R³, R⁵ und für eine Verbindung jeweils einer Zeile der Tabelle A entspricht

### Tabelle 94

Verbindungen der allgemeinen Formel I-A, in denen n für 1, R² für 3-Methyl, R⁴ für Ethyl und R⁶ für Ethoxy steht und die Kombination der Reste R¹, R³ und R⁵ für eine Verbindung jeweils einer Zeile der Tabelle A entspricht

### Tabelle 95

Verbindungen der allgemeinen Formel I-A, in denen n für 1, R² für 5-Methyl, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 96

Verbindungen der allgemeinen Formel I-A, in denen n für 1, R² für 3-Trifluormethyl, R4 für Ethyl und R⁶ für Ethoxy steht und die Kombination der Reste R¹, R³ und R⁵ für eine Verbindung jeweils einer Zeile der Tabelle A entspricht

### Tabelle 97

Verbindungen der allgemeinen Formel I-A, in denen n für 1, R² für 5-Trifluormethyl, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 98

Verbindungen der allgemeinen Formel I-A, in denen n für 1, R² für 3-Hydroxy, R⁴ für Ethyl und R⁶ für Ethoxy steht und die Kombination der Reste R¹, R³ und R⁵ für eine Verbindung jeweils einer Zeile der Tabelle A entspricht

### Tabelle 99

Verbindungen der allgemeinen Formel I-A, in denen n für 1, R² für 5-Hydroxy, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 100

Verbindungen der allgemeinen Formel I-A, in denen n für 1, R² für 3-Methoxy, R⁴ für Ethyl und R⁶ für Ethoxy steht und die Kombination der Reste R¹, R³ und R⁵ für eine Verbindung jeweils einer Zeile der Tabelle A entspricht

### Tabelle 101

Verbindungen der allgemeinen Formel I-A, in denen n für 1, R² für 5-Methoxy, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 102

Verbindungen der allgemeinen Formel I-A, in denen n für 1, R² für 3-Ethoxy, R⁴ für Ethyl und R⁶ für Ethoxy steht und die Kombination der Reste R¹, R³ und R⁵ für eine Verbindung jeweils einer Zeile der Tabelle A entspricht

### Tabelle 103

Verbindungen der allgemeinen Formel I-A, in denen n für 1, R² für 5-Ethoxy, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 104

Verbindungen der allgemeinen Formel I-A, in denen n für 1, R² für 3-Difluormethoxy, R⁴ für Ethyl und R⁶ für Ethoxy steht und die Kombination der Reste R¹, R³ und R⁵ für eine Verbindung jeweils einer Zeile der Tabelle A entspricht

### Tabelle 105

Verbindungen der allgemeinen Formel I-A, in denen n für 1, R² für 5-Difluormethoxy, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 106

Verbindungen der allgemeinen Formel I-A, in denen n für 1, R² für 3-Chlorfluormethoxy, R⁴ für Ethyl und R⁶ für Ethoxy steht und die Kombination der Reste R¹, R³ und R⁵ für eine Verbindung jeweils einer Zeile der Tabelle A entspricht

### Tabelle 107

Verbindungen der allgemeinen Formel I-A, in denen n für 1, R² für 5-Chlorfluormethoxy, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 108

Verbindungen der allgemeinen Formel I-A, in denen n für 1, R² für 3-Fluor, R⁴ für Ethyl und R⁶ für Ethoxy steht und die Kombination der Reste R¹, R³ und R⁵ für eine Verbindung jeweils einer Zeile der Tabelle A entspricht

### Tabelle 109

Verbindungen der allgemeinen Formel I-A, in denen n für 1, R² für 5-Fluor, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 110

Verbindungen der allgemeinen Formel I-A, in denen n für 1, R² für 3-Chlor, R⁴ für Ethyl und R⁶ für Ethoxy steht und die Kombination der Reste R¹, R³ und R⁵ für eine Verbindung jeweils einer Zeile der Tabelle A entspricht

### Tabelle 111

Verbindungen der allgemeinen Formel I-A in denen n für 1, R² für 5-Chlor, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 112

Verbindungen der allgemeinen Formel I-A, in denen n für 1, R² für 3-Brom, R⁴ für Ethyl und R⁶ für Ethoxy steht und die Kombination der Reste R¹, R³ und R⁵ für eine Verbindung jeweils einer Zeile der Tabelle A entspricht

### Tabelle 113

Verbindungen der allgemeinen Formel I-A, in denen n für 1, R² für 5-Brom, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 114

Verbindungen der allgemeinen Formel I-A, in denen n für 1, R² für 3-Iod, R⁴ für Ethyl und R⁶ für Ethoxy steht und die Kombination der Reste R¹, R³ und R⁵ für eine Verbindung jeweils einer Zeile der Tabelle A entspricht

### Tabelle 115

Verbindungen der allgemeinen Formel I-A, in denen n für 1, R² für 5-Iod, R⁴ für Ethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 116

Verbindungen der allgemeinen Formel I-A, in denen n 0 ist, R⁴ für Trifluormethyl und R⁶ für Ethoxy steht und die Kombination der Reste R¹, R³, R⁵ und für eine Verbindung jeweils einer Zeile der Tabelle A entspricht

### Tabelle 117

Verbindungen der allgemeinen Formel I-A, in denen n für 1, R² für 3-Methyl, R⁴ für Trifluormethyl und R⁶ für Ethoxy steht und die, Kombination der Reste R¹, R³ und R⁵ für eine Verbindung jeweils einer Zeile der Tabelle A entspricht

### Tabelle 118

Verbindungen der allgemeinen Formel I-A, in denen n für 1, R² für 5-Methyl, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 119

Verbindungen der allgemeinen Formel I-A, in denen n für 1, R² für 3-Trifluormethyl, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 120

Verbindungen der allgemeinen Formel I-A, in denen n für 1, R² für 5-Trifluormethyl, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 121

Verbindungen der allgemeinen Formel I-A, in denen n für 1, R² für 3-Hydroxy, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 122

Verbindungen der allgemeinen Formel I-A, in denen n für 1, R² für 5-Hydroxy, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 123

Verbindungen der allgemeinen Formel I-A, in denen n für 1, R² für 3-Methoxy, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 124

Verbindungen der allgemeinen Formel I-A, in denen n für 1, R² für 5-Methoxy, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 125

Verbindungen der allgemeinen Formel I-A, in denen n für 1, R² für 3-Ethoxy, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 126

Verbindungen der allgemeinen Formel I-A, in denen n für 1, R² für 5-Ethoxy, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R3 ist

### Tabelle 127

Verbindungen der allgemeinen Formel I-A, in denen n für 1, R² für 3-Difluormethoxy, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 128

Verbindungen der allgemeinen Formel I-A, in denen n für 1, R² für 5-Difluormethoxy, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 129

Verbindungen der allgemeinen Formel I-A, in denen n für 1, R² für 3-Chlorfluormethoxy, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 130

Verbindungen der allgemeinen Formel I-A, in denen n für 1, R² für 5-Chlorfluormethoxy, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 131

Verbindungen der allgemeinen Formel I-A, in denen n für 1, R² für 3-Fluor, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 132

Verbindungen der allgemeinen Formel I-A, in denen n für 1, R² für 5-Fluor, R⁴ für Trifluormethyl und R⁶ für Ethoxy steht und die Kombination der Reste R¹, R³ und R⁵ für eine Verbindung jeweils, einer Zeile der Tabelle A entspricht, wobei R¹ ungleich R³ ist

### Tabelle 133

Verbindungen der allgemeinen Formel I-A, in denen n für 1, R² für 3-Chlor, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 134

Verbindungen der allgemeinen Formel I-A, in denen n für 1, R² für 5-Chlor, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 135

Verbindungen der allgemeinen Formel I-A, in denen n für 1, R² für 3-Brom, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 136

Verbindungen der allgemeinen Formel I-A, in denen n für 1, R² für 5-Brom, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

### Tabelle 137

Verbindungen der allgemeinen Formel I-A, in denen n für 1, R² für 3-Iod, R⁴ für Trifluormethyl und R⁶ für Ethoxy steht und die Kombination der Reste R¹, R³ und R⁵ für eine Verbindung jeweils einer Zeile der Tabelle A entspricht

### Tabelle 138

Verbindungen der allgemeinen Formel I-A, in denen n für 1, R² für 5-Iod, R⁴ für Trifluormethyl und R⁶ für Ethoxy steht und die Kombination der Reste R¹, R³ und R⁵ für eine Verbindung jeweils einer Zeile der Tabelle A entspricht, wobei R¹ ungleich R³ ist

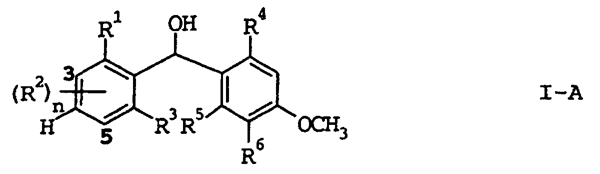

**Tabelle A**

| **Nr.** | **R**^{**1**} | **R**^{**3**} | **R**^{**5**} |
|---|---|---|---|
| A-1 | CH₃ | CH₃ | OH |
| A-2 | CH₂CH₃ | CH₃ | OH |
| A-3 | CF₃ | CH₃ | OH |
| A-4 | F | CH₃ | OH |
| A-5 | Cl | CH₃ | OH |
| A-6 | Br | CH₃ | OH |
| A-7 | I | CH₃ | OH |
| A-8 | OH | CH₃ | OH |
| A-9 | SH | CH₃ | OH |
| A-10 | SCH₃ | CH₃ | OH |
| A-11 | NH₂ | CH₃ | OH |
| A-12 | OCH₃ | CH₃ | OH |
| A-13 | OCHF₂ | CH₃ | OH |
| A-14 | OCHFCl | CH₃ | OH |
| A-15 | OCF₃ | CH₃ | OH |
| A-16 | OC(=O)CH₃ | CH₃ | OH |
| A-17 | OC(=O)CH₂CH₃ | CH₃ | OH |
| A-18 | CH₂CH₃ | CH₂CH₃ | OH |
| A-19 | CF₃ | CH₂CH₃ | OH |
| A-20 | F | CH₂CH₃ | OH |
| A-21 | Cl | CH₂CH₃ | OH |
| A-22 | Br | CH₂CH₃ | OH |
| A-23 | I | CH₂CH₃ | OH |
| A-24 | OH | CH₂CH₃ | OH |
| A-25 | SH | CH₂CH₃ | OH |
| A-26 | SCH₃ | CH₂CH₃ | OH |
| A-27 | NH₂ | CH₂CH₃ | OH |
| A-28 | OCH₃ | CH₂CH₃ | OH |
| A-29 | OCHF₂ | CH₂CH₃ | OH |
| A-30 | OCHFCl | CH₂CH₃ | OH |
| A-31 | OCF₃ | CH₂CH₃ | OH |
| A-32 | OC(=O)CH₃ | CH₂CH₃ | OH |
| A-33 | OC(=O)CH₂CH₃ | CH₂CH₃ | OH |
| A-34 | CF₃ | CF₃ | OH |
| A-35 | F | CF₃ | OH |
| A-36 | Cl | CF₃ | OH |
| A-37 | Br | CF₃ | OH |
| A-38 | I | CF₃ | OH |
| A-39 | OH | CF₃ | OH |
| A-40 | SH | CF₃ | OH |
| A-41 | SCH₃ | CF₃ | OH |
| A-42 | NH₂ | CF₃ | OH |
| A-43 | OCH₃ | CF₃ | OH |
| A-44 | OCHF₂ | CF₃ | OH |
| A-45 | OCHFCl | CF₃ | OH |
| A-46 | OCF₃ | CF₃ | OH |
| A-47 | OC(=O)CH₃ | CF₃ | OH |
| A-48 | OC(=O)CH₂CH₃ | CF₃ | OH |
| A-49 | F | F | OH |
| A-50 | Cl | F | OH |
| A-51 | Br | F | OH |
| A-52 | I | F | OH |
| A-53 | OH | F | OH |
| A-54 | SH | F | OH |
| A-55 | SCH₃ | F | OH |
| A-56 | NH₂ | F | OH |
| A-57 | OCH₃ | F | OH |
| A-58 | OCHF₂ | F | OH |
| A-59 | OCHFCl | F | OH |
| A-60 | OCF₃ | F | OH |
| A-61 | OC(=O)CH₃ | F | OH |
| A-62 | OC(=O)CH₂CH₃ | F | OH |
| A-63 | Cl | Cl | OH |
| A-64 | Br | Cl | OH |
| A-65 | I | Cl | OH |
| A-66 | OH | Cl | OH |
| A-67 | SH | Cl | OH |
| A-68 | SCH₃ | Cl | OH |
| A-69 | NH₂ | Cl | OH |
| A-70 | OCH₃ | Cl | OH |
| A-71 | OCHF₂ | Cl | OH |
| A-72 | OCHFCl | Cl | OH |
| A-73 | OCF₃ | Cl | OH |
| A-74 | OC(=O)CH₃ | Cl | OH |
| A-75 | OC(=O)CH₂CH₃ | Cl | OH |
| A-76 | Br | Br | OH |
| A-77 | I | Br | OH |
| A-78 | OH | Br | OH |
| A-79 | SH | Br | OH |
| A-80 | SCH₃ | Br | OH |
| A-81 | NH₂ | Br | OH |
| A-82 | OCH₃ | Br | OH |
| A-83 | OCHF₂ | Br | OH |
| A-84 | OCHFCl | Br | OH |
| A-85 | OCF₃ | Br | OH |
| A-86 | OC(=O)CH₃ | Br | OH |
| A-87 | OC(=O)CH₂CH₃ | Br | OH |
| A-88 | I | I | OH |
| A-89 | OH | I | OH |
| A-90 | SH | I | OH |
| A-91 | SCH₃ | I | OH |
| A-92 | NH₂ | I | OH |
| A-93 | OCH₃ | I | OH |
| A-94 | OCHF₂ | I | OH |
| A-95 | OCHFCl | I | OH |
| A-96 | OCF₃ | I | OH |
| A-97 | OC(=O)CH₃ | I | OH |
| A-98 | OC(=O)CH₂CH₃ | I | OH |
| A-99 | OH | OH | OH |
| A-100 | SH | OH | OH |
| A-101 | SCH₃ | OH | OH |
| A-102 | NH₂ | OH | OH |
| A-103 | OCH₃ | OH | OH |
| A-104 | OCHF₂ | OH | OH |
| A-105 | OCHFCl | OH | OH |
| A-106 | OCF₃ | OH | OH |
| A-107 | OC(=O)CH₃ | OH | OH |
| A-108 | OC(=O)CH₂CH₃ | OH | OH |
| A-109 | SH | SH | OH |
| A-110 | SCH₃ | SH | OH |
| A-111 | NH₂ | SH | OH |
| A-112 | OCH₃ | SH | OH |
| A-113 | OCHF₂ | SH | OH |
| A-114 | OCHFCl | SH | OH |
| A-115 | OCF₃ | SH | OH |
| A-116 | OC(=O)CH₃ | SH | OH |
| A-117 | OC(=O)CH₂CH₃ | SH | OH |
| A-118 | SCH₃ | SCH₃ | OH |
| A-119 | NH₂ | SCH₃ | OH |
| A-120 | OCH₃ | SCH₃ | OH |
| A-121 | OCHF₂ | SCH₃ | OH |
| A-122 | OCHFCl | SCH₃ | OH |
| A-123 | OCF₃ | SCH₃ | OH |
| A-124 | OC(=O)CH₃ | SCH₃ | OH |
| A-125 | OC(=O)CH₂CH₃ | SCH₃ | OH |
| A-126 | NH₂ | NH₂ | OH |
| A-127 | OCH₃ | NH₂ | OH |
| A-128 | OCHF₂ | NH₂ | OH |
| A-129 | OCHFCl | NH₂ | OH |
| A-130 | OCF₃ | NH₂ | OH |
| A-131 | OC(=O)CH₃ | NH₂ | OH |
| A-132 | OC(=O)CH₂CH₃ | NH₂ | OH |
| A-133 | OCH₃ | OCH₃ | OH |
| A-134 | OCHF₂ | OCH₃ | OH |
| A-135 | OCHFCl | OCH₃ | OH |
| A-136 | OCF₃ | OCH₃ | OH |
| A-137 | OC(=O)CH₃ | OCH₃ | OH |
| A-138 | OC(=O)CH₂CH₃ | OCH₃ | OH |
| A-139 | OCHF₂ | OCHF₂ | OH |
| A-140 | OCHFCl | OCHF₂ | OH |
| A-141 | OCF₃ | OCHF₂ | OH |
| A-142 | OC(=O)CH₃ | OCHF₂ | OH |
| A-143 | OC(=O)CH₂CH₃ | OCHF₂ | OH |
| A-144 | OCHFCl | OCHFCl | OH |
| A-145 | OCF₃ | OCHFCl | OH |
| A-146 | OC(=O)CH₃ | OCHFCl | OH |
| A-147 | OC(=O)CH₂CH₃ | OCHFCl | OH |
| A-148 | OCF₃ | OCF₃ | OH |
| A-149 | OC(=O)CH₃ | OCF₃ | OH |
| A-150 | OC(=O)CH₂CH₃ | OCF₃ | OH |
| A-151 | OC(=O)CH₃ | OC(=O)CH₃ | OH |
| A-152 | OC(=O)CH₂CH₃ | OC(=O)CH₃ | OH |
| A-153 | OC(=O)CH₂CH₃ | OC(=O)CH₂CH₃ | OH |
| A-154 | CH₃ | CH₃ | OCH₃ |
| A-155 | CH₂CH₃ | CH₃ | OCH₃ |
| A-156 | CF₃ | CH₃ | OCH₃ |
| A-157 | F | CH₃ | OCH₃ |
| A-158 | Cl | CH₃ | OCH₃ |
| A-159 | Br | CH₃ | OCH₃ |
| A-160 | I | CH₃ | OCH₃ |
| A-161 | OH | CH₃ | OCH₃ |
| A-162 | SH | CH₃ | OCH₃ |
| A-163 | SCH₃ | CH₃ | OCH₃ |
| A-164 | NH₂ | CH₃ | OCH₃ |
| A-165 | OCH₃ | CH₃ | OCH₃ |
| A-166 | OCHF₂ | CH₃ | OCH₃ |
| A-167 | OCHFCl | CH₃ | OCH₃ |
| A-168 | OCF₃ | CH₃ | OCH₃ |
| A-169 | OC(=O)CH₃ | CH₃ | OCH₃ |
| A-170 | OC(=O)CH₂CH₃ | CH₃ | OCH₃ |
| A-171 | CH₂CH₃ | CH₂CH₃ | OCH₃ |
| A-172 | CF₃ | CH₂CH₃ | OCH₃ |
| A-173 | F | CH₂CH₃ | OCH₃ |
| A-174 | Cl | CH₂CH₃ | OCH₃ |
| A-175 | Br | CH₂CH₃ | OCH₃ |
| A-176 | I | CH₂CH₃ | OCH₃ |
| A-177 | OH | CH₂CH₃ | OCH₃ |
| A-178 | SH | CH₂CH₃ | OCH₃ |
| A-179 | SCH₃ | CH₂CH₃ | OCH₃ |
| A-180 | NH₂ | CH₂CH₃ | OCH₃ |
| A-181 | OCH₃ | CH₂CH₃ | OCH₃ |
| A-182 | OCHF₂ | CH₂CH₃ | OCH₃ |
| A-183 | OCHFCl | CH₂CH₃ | OCH₃ |
| A-184 | OCF₃ | CH₂CH₃ | OCH₃ |
| A-185 | OC(=O)CH₃ | CH₂CH₃ | OCH₃ |
| A-186 | OC(=O)CH₂CH₃ | CH₂CH₃ | OCH₃ |
| A-187 | CF₃ | CF₃ | OCH₃ |
| A-188 | F | CF₃ | OCH₃ |
| A-189 | Cl | CF₃ | OCH₃ |
| A-190 | Br | CF₃ | OCH₃ |
| A-191 | I | CF₃ | OCH₃ |
| A-192 | OH | CF₃ | OCH₃ |
| A-193 | SH | CF₃ | OCH₃ |
| A-194 | SCH₃ | CF₃ | OCH₃ |
| A-195 | NH₂ | CF₃ | OCH₃ |
| A-196 | OCH₃ | CF₃ | OCH₃ |
| A-197 | OCHF₂ | CF₃ | OCH₃ |
| A-198 | OCHFCl | CF₃ | OCH₃ |
| A-199 | OCF₃ | CF₃ | OCH₃ |
| A-200 | OC(=O)CH₃ | CF₃ | OCH₃ |
| A-201 | OC(=O)CH₂CH₃ | CF₃ | OCH₃ |
| A-202 | F | F | OCH₃ |
| A-203 | Cl | F | OCH₃ |
| A-204 | Br | F | OCH₃ |
| A-205 | I | F | OCH₃ |
| A-206 | OH | F | OCH₃ |
| A-207 | SH | F | OCH₃ |
| A-208 | SCH₃ | F | OCH₃ |
| A-209 | NH₂ | F | OCH₃ |
| A-210 | OCH₃ | F | OCH₃ |
| A-211 | OCHF₂ | F | OCH₃ |
| A-212 | OCHFCl | F | OCH₃ |
| A-213 | OCF₃ | F | OCH₃ |
| A-214 | OC(=O)CH₃ | F | OCH₃ |
| A-215 | OC(=O)CH₂CH₃ | F | OCH₃ |
| A-216 | Cl | Cl | OCH₃ |
| A-217 | Br | Cl | OCH₃ |
| A-218 | I | Cl | OCH₃ |
| A-219 | OH | Cl | OCH₃ |
| A-220 | SH | Cl | OCH₃ |
| A-221 | SCH₃ | Cl | OCH₃ |
| A-222 | NH₂ | Cl | OCH₃ |
| A-223 | OCH₃ | Cl | OCH₃ |
| A-224 | OCHF₂ | Cl | OCH₃ |
| A-225 | OCHFCl | Cl | OCH₃ |
| A-226 | OCF₃ | Cl | OCH₃ |
| A-227 | OC(=O)CH₃ | Cl | OCH₃ |
| A-228 | OC(=O)CH₂CH₃ | Cl | OCH₃ |
| A-229 | Br | Br | OCH₃ |
| A-230 | I | Br | OCH₃ |
| A-231 | OH | Br | OCH₃ |
| A-232 | SH | Br | OCH₃ |
| A-233 | SCH₃ | Br | OCH₃ |
| A-234 | NH₂ | Br | OCH₃ |
| A-235 | OCH₃ | Br | OCH₃ |
| A-236 | OCHF₂ | Br | OCH₃ |
| A-237 | OCHFCl | Br | OCH₃ |
| A-238 | OCF₃ | Br | OCH₃ |
| A-239 | OC(=O)CH₃ | Br | OCH₃ |
| A-240 | OC(=O)CH₂CH₃ | Br | OCH₃ |
| A-241 | I | I | OCH₃ |
| A-242 | OH | I | OCH₃ |
| A-243 | SH | I | OCH₃ |
| A-244 | SCH₃ | I | OCH₃ |
| A-245 | NH₂ | I | OCH₃ |
| A-246 | OCH₃ | I | OCH₃ |
| A-247 | OCHF₂ | I | OCH₃ |
| A-248 | OCHFCl | I | OCH₃ |
| A-249 | OCF₃ | I | OCH₃ |
| A-250 | OC(=O)CH₃ | I | OCH₃ |
| A-251 | OC(=O)CH₂CH₃ | I | OCH₃ |
| A-252 | OH | OH | OCH₃ |
| A-253 | SH | OH | OCH₃ |
| A-254 | SCH₃ | OH | OCH₃ |
| A-255 | NH₂ | OH | OCH₃ |
| A-256 | OCH₃ | OH | OCH₃ |
| A-257 | OCHF₂ | OH | OCH₃ |
| A-258 | OCHFCl | OH | OCH₃ |
| A-259 | OCF₃ | OH | OCH₃ |
| A-260 | OC(=O)CH₃ | OH | OCH₃ |
| A-261 | OC(=O)CH₂CH₃ | OH | OCH₃ |
| A-262 | SH | SH | OCH₃ |
| A-263 | SCH₃ | SH | OCH₃ |
| A-264 | NH₂ | SH | OCH₃ |
| A-265 | OCH₃ | SH | OCH₃ |
| A-266 | OCHF₂ | SH | OCH₃ |
| A-267 | OCHFCl | SH | OCH₃ |
| A-268 | OCF₃ | SH | OCH₃ |
| A-269 | OC(=O)CH₃ | SH | OCH₃ |
| A-270 | OC(=O)CH₂CH₃ | SH | OCH₃ |
| A-271 | SCH₃ | SCH₃ | OCH₃ |
| A-272 | NH₂ | SCH₃ | OCH₃ |
| A-273 | OCH₃ | SCH₃ | OCH₃ |
| A-274 | OCHF₂ | SCH₃ | OCH₃ |
| A-275 | OCHFCl | SCH₃ | OCH₃ |
| A-276 | OCF₃ | SCH₃ | OCH₃ |
| A-277 | OC(=O)CH₃ | SCH₃ | OCH₃ |
| A-278 | OC(=O)CH₂CH₃ | SCH₃ | OCH₃ |
| A-279 | NH₂ | NH₂ | OCH₃ |
| A-280 | OCH₃ | NH₂ | OCH₃ |
| A-281 | OCHF₂ | NH₂ | OCH₃ |
| A-282 | OCHFCl | NH₂ | OCH₃ |
| A-283 | OCF₃ | NH₂ | OCH₃ |
| A-284 | OC(=O)CH₃ | NH₂ | OCH₃ |
| A-285 | OC(=O)CH₂CH₃ | NH₂ | OCH₃ |
| A-286 | OCH₃ | OCH₃ | OCH₃ |
| A-287 | OCHF₂ | OCH₃ | OCH₃ |
| A-288 | OCHFCl | OCH₃ | OCH₃ |
| A-289 | OCF₃ | OCH₃ | OCH₃ |
| A-290 | OC(=O)CH₃ | OCH₃ | OCH₃ |
| A-291 | OC(=O)CH₂CH₃ | OCH₃ | OCH₃ |
| A-292 | OCHF₂ | OCHF₂ | OCH₃ |
| A-293 | OCHFCl | OCHF₂ | OCH₃ |
| A-294 | OCF₃ | OCHF₂ | OCH₃ |
| A-295 | OC(=O)CH₃ | OCHF₂ | OCH₃ |
| A-296 | OC(=O)CH₂CH₃ | OCHF₂ | OCH₃ |
| A-297 | OCHFCl | OCHFCl | OCH₃ |
| A-298 | OCF₃ | OCHFCl | OCH₃ |
| A-299 | OC(=O)CH₃ | OCHFCl | OCH₃ |
| A-300 | OC(=O)CH₂CH₃ | OCHFCl | OCH₃ |
| A-301 | OCF₃ | OCF₃ | OCH₃ |
| A-302 | OC(=O)CH₃ | OCF₃ | OCH₃ |
| A-303 | OC(=O)CH₂CH₃ | OCF₃ | OCH₃ |
| A-304 | OC(=O)CH₃ | OC(=O)CH₃ | OCH₃ |
| A-305 | OC(=O)CH₂CH₃ | OC(=O)CH₃ | OCH₃ |
| A-306 | OC(=O)CH₂CH₃ | OC(=O)CH₂CH₃ | OCH₃ |
| A-307 | CH₃ | CH₃ | OCH₂CH₃ |
| A-308 | CH₂CH₃ | CH₃ | OCH₂CH₃ |
| A-309 | CF₃ | CH₃ | OCH₂CH₃ |
| A-310 | F | CH₃ | OCH₂CH₃ |
| A-311 | Cl | CH₃ | OCH₂CH₃ |
| A-312 | Br | CH₃ | OCH₂CH₃ |
| A-313 | I | CH₃ | OCH₂CH₃ |
| A-314 | OH | CH₃ | OCH₂CH₃ |
| A-315 | SH | CH₃ | OCH₂CH₃ |
| A-316 | SCH₃ | CH₃ | OCH₂CH₃ |
| A-317 | NH₂ | CH₃ | OCH₂CH₃ |
| A-318 | OCH₃ | CH₃ | OCH₂CH₃ |
| A-319 | OCHF₂ | CH₃ | OCH₂CH₃ |
| A-320 | OCHFCl | CH₃ | OCH₂CH₃ |
| A-321 | OCF₃ | CH₃ | OCH₂CH₃ |
| A-322 | OC(=O)CH₃ | CH₃ | OCH₂CH₃ |
| A-323 | OC(=O)CH₂CH₃ | CH₃ | OCH₂CH₃ |
| A-324 | CH₂CH₃ | CH₂CH₃ | OCH₂CH₃ |
| A-325 | CF₃ | CH₂CH₃ | OCH₂CH₃ |
| A-326 | F | CH₂CH₃ | OCH₂CH₃ |
| A-327 | Cl | CH₂CH₃ | OCH₂CH₃ |
| A-328 | Br | CH₂CH₃ | OCH₂CH₃ |
| A-329 | I | CH₂CH₃ | OCH₂CH₃ |
| A-330 | OH | CH₂CH₃ | OCH₂CH₃ |
| A-331 | SH | CH₂CH₃ | OCH₂CH₃ |
| A-332 | SCH₃ | CH₂CH₃ | OCH₂CH₃ |
| A-333 | NH₂ | CH₂CH₃ | OCH₂CH₃ |
| A-334 | OCH₃ | CH₂CH₃ | OCH₂CH₃ |
| A-335 | OCHF₂ | CH₂CH₃ | OCH₂CH₃ |
| A-336 | OCHFCl | CH₂CH₃ | OCH₂CH₃ |
| A-337 | OCF₃ | CH₂CH₃ | OCH₂CH₃ |
| A-338 | OC(=O)CH₃ | CH₂CH₃ | OCH₂CH₃ |
| A-339 | OC(=O)CH₂CH₃ | CH₂CH₃ | OCH₂CH₃ |
| A-340 | CF₃ | CF₃ | OCH₂CH₃ |
| A-341 | F | CF₃ | OCH₂CH₃ |
| A-342 | Cl | CF₃ | OCH₂CH₃ |
| A-343 | Br | CF₃ | OCH₂CH₃ |
| A-344 | I | CF₃ | OCH₂CH₃ |
| A-345 | OH | CF₃ | OCH₂CH₃ |
| A-346 | SH | CF₃ | OCH₂CH₃ |
| A-347 | SCH₃ | CF₃ | OCH₂CH₃ |
| A-348 | NH₂ | CF₃ | OCH₂CH₃ |
| A-349 | OCH₃ | CF₃ | OCH₂CH₃ |
| A-350 | OCHF₂ | CF₃ | OCH₂CH₃ |
| A-351 | OCHFCl | CF₃ | OCH₂CH₃ |
| A-352 | OCF₃ | CF₃ | OCH₂CH₃ |
| A-353 | OC(=O)CH₃ | CF₃ | OCH₂CH₃ |
| A-354 | OC(=O)CH₂CH₃ | CF₃ | OCH₂CH₃ |
| A-355 | F | F | OCH₂CH₃ |
| A-356 | Cl | F | OCH₂CH₃ |
| A-357 | Br | F | OCH₂CH₃ |
| A-358 | I | F | OCH₂CH₃ |
| A-359 | OH | F | OCH₂CH₃ |
| A-360 | SH | F | OCH₂CH₃ |
| A-361 | SCH₃ | F | OCH₂CH₃ |
| A-362 | NH₂ | F | OCH₂CH₃ |
| A-363 | OCH₃ | F | OCH₂CH₃ |
| A-364 | OCHF₂ | F | OCH₂CH₃ |
| A-365 | OCHFCl | F | OCH₂CH₃ |
| A-366 | OCF₃ | F | OCH₂CH₃ |
| A-367 | OC(=O)CH₃ | F | OCH₂CH₃ |
| A-368 | OC(=O)CH₂CH₃ | F | OCH₂CH₃ |
| A-369 | Cl | Cl | OCH₂CH₃ |
| A-370 | Br | Cl | OCH₂CH₃ |
| A-371 | I | Cl | OCH₂CH₃ |
| A-372 | OH | Cl | OCH₂CH₃ |
| A-373 | SH | Cl | OCH₂CH₃ |
| A-374 | SCH₃ | Cl | OCH₂CH₃ |
| A-375 | NH₂ | Cl | OCH₂CH₃ |
| A-376 | OCH₃ | Cl | OCH₂CH₃ |
| A-377 | OCHF₂ | Cl | OCH₂CH₃ |
| A-378 | OCHFCl | Cl | OCH₂CH₃ |
| A-379 | OCF₃ | Cl | OCH₂CH₃ |
| A-380 | OC(=O)CH₃ | Cl | OCH₂CH₃ |
| A-381 | OC(=O)CH₂CH₃ | Cl | OCH₂CH₃ |
| A-382 | Br | Br | OCH₂CH₃ |
| A-383 | I | Br | OCH₂CH₃ |
| A-384 | OH | Br | OCH₂CH₃ |
| A-385 | SH | Br | OCH₂CH₃ |
| A-386 | SCH₃ | Br | OCH₂CH₃ |
| A-387 | NH₂ | Br | OCH₂CH₃ |
| A-388 | OCH₃ | Br | OCH₂CH₃ |
| A-389 | OCHF₂ | Br | OCH₂CH₃ |
| A-390 | OCHFCl | Br | OCH₂CH₃ |
| A-391 | OCF₃ | Br | OCH₂CH₃ |
| A-392 | OC(=O)CH₃ | Br | OCH₂CH₃ |
| A-393 | OC(=O)CH₂CH₃ | Br | OCH₂CH₃ |
| A-394 | I | I | OCH₂CH₃ |
| A-395 | OH | I | OCH₂CH₃ |
| A-396 | SH | I | OCH₂CH₃ |
| A-397 | SCH₃ | I | OCH₂CH₃ |
| A-398 | NH₂ | I | OCH₂CH₃ |
| A-399 | OCH₃ | I | OCH₂CH₃ |
| A-400 | OCHF₂ | I | OCH₂CH₃ |
| A-401 | OCHFCl | I | OCH₂CH₃ |
| A-402 | OCF₃ | I | OCH₂CH₃ |
| A-403 | OC(=O)CH₃ | I | OCH₂CH₃ |
| A-404 | OC(=O)CH₂CH₃ | I | OCH₂CH₃ |
| A-405 | OH | OH | OCH₂CH₃ |
| A-406 | SH | OH | OCH₂CH₃ |
| A-407 | SCH₃ | OH | OCH₂CH₃ |
| A-408 | NH₂ | OH | OCH₂CH₃ |
| A-409 | OCH₃ | OH | OCH₂CH₃ |
| A-410 | OCHF₂ | OH | OCH₂CH₃ |
| A-411 | OCHFCl | OH | OCH₂CH₃ |
| A-412 | OCF₃ | OH | OCH₂CH₃ |
| A-413 | OC(=O)CH₃ | OH | OCH₂CH₃ |
| A-414 | OC(=O)CH₂CH₃ | OH | OCH₂CH₃ |
| A-415 | SH | SH | OCH₂CH₃ |
| A-416 | SCH₃ | SH | OCH₂CH₃ |
| A-417 | NH₂ | SH | OCH₂CH₃ |
| A-418 | OCH₃ | SH | OCH₂CH₃ |
| A-419 | OCHF₂ | SH | OCH₂CH₃ |
| A-420 | OCHFCl | SH | OCH₂CH₃ |
| A-421 | OCF₃ | SH | OCH₂CH₃ |
| A-422 | OC(=O)CH₃ | SH | OCH₂CH₃ |
| A-423 | OC(=O)CH₂CH₃ | SH | OCH₂CH₃ |
| A-424 | SCH₃ | SCH₃ | OCH₂CH₃ |
| A-425 | NH₂ | SCH₃ | OCH₂CH₃ |
| A-426 | OCH₃ | SCH₃ | OCH₂CH₃ |
| A-427 | OCHF₂ | SCH₃ | OCH₂CH₃ |
| A-428 | OCHFCl | SCH₃ | OCH₂CH₃ |
| A-429 | OCF₃ | SCH₃ | OCH₂CH₃ |
| A-430 | OC(=O)CH₃ | SCH₃ | OCH₂CH₃ |
| A-431 | OC(=O)CH₂CH₃ | SCH₃ | OCH₂CH₃ |
| A-432 | NH₂ | NH₂ | OCH₂CH₃ |
| A-433 | OCH₃ | NH₂ | OCH₂CH₃ |
| A-434 | OCHF₂ | NH₂ | OCH₂CH₃ |
| A-435 | OCHFCl | NH₂ | OCH₂CH₃ |
| A-436 | OCF₃ | NH₂ | OCH₂CH₃ |
| A-437 | OC(=O)CH₃ | NH₂ | OCH₂CH₃ |
| A-438 | OC(=O)CH₂CH₃ | NH₂ | OCH₂CH₃ |
| A-439 | OCH₃ | OCH₃ | OCH₂CH₃ |
| A-440 | OCHF₂ | OCH₃ | OCH₂CH₃ |
| A-441 | OCHFCl | OCH₃ | OCH₂CH₃ |
| A-442 | OCF₃ | OCH₃ | OCH₂CH₃ |
| A-443 | OC(=O)CH₃ | OCH₃ | OCH₂CH₃ |
| A-444 | OC(=O)CH₂CH₃ | OCH₃ | OCH₂CH₃ |
| A-445 | OCHF₂ | OCHF₂ | OCH₂CH₃ |
| A-446 | OCHFCl | OCHF₂ | OCH₂CH₃ |
| A-447 | OCF₃ | OCHF₂ | OCH₂CH₃ |
| A-448 | OC(=O)CH₃ | OCHF₂ | OCH₂CH₃ |
| A-449 | OC(=O)CH₂CH₃ | OCHF₂ | OCH₂CH₃ |
| A-450 | OCHFCl | OCHFCl | OCH₂CH₃ |
| A-451 | OCF₃ | OCHFCl | OCH₂CH₃ |
| A-452 | OC(=O)CH₃ | OCHFCl | OCH₂CH₃ |
| A-453 | OC(=O)CH₂CH₃ | OCHFCl | OCH₂CH₃ |
| A-454 | OCF₃ | OCF₃ | OCH₂CH₃ |
| A-455 | OC(=O)CH₃ | OCF₃ | OCH₂CH₃ |
| A-456 | OC(=O)CH₂CH₃ | OCF₃ | OCH₂CH₃ |
| A-457 | OC(=O)CH₃ | OC(=O)CH₃ | OCH₂CH₃ |
| A-458 | OC(=O)CH₂CH₃ | OC(=O)CH₃ | OCH₂CH₃ |
| A-459 | OC(=O)CH₂CH₃ | OC(=O)CH₂CH₃ | OCH₂CH₃ |
| A-460 | CH₃ | CH₃ | OCH₂CH₂CH₃ |
| A-461 | CH₂CH₃ | CH₃ | OCH₂CH₂CH₃ |
| A-462 | CF₃ | CH₃ | OCH₂CH₂CH₃ |
| A-463 | F | CH₃ | OCH₂CH₂CH₃ |
| A-464 | Cl | CH₃ | OCH₂CH₂CH₃ |
| A-465 | Br | CH₃ | OCH₂CH₂CH₃ |
| A-466 | I | CH₃ | OCH₂CH₂CH₃ |
| A-467 | OH | CH₃ | OCH₂CH₂CH₃ |
| A-468 | SH | CH₃ | OCH₂CH₂CH₃ |
| A-469 | SCH₃ | CH₃ | OCH₂CH₂CH₃ |
| A-470 | NH₂ | CH₃ | OCH₂CH₂CH₃ |
| A-471 | OCH₃ | CH₃ | OCH₂CH₂CH₃ |
| A-472 | OCHF₂ | CH₃ | OCH₂CH₂CH₃ |
| A-473 | OCHFCl | CH₃ | OCH₂CH₂CH₃ |
| A-474 | OCF₃ | CH₃ | OCH₂CH₂CH₃ |
| A-475 | OC(=O)CH₃ | CH₃ | OCH₂CH₂CH₃ |
| A-476 | OC(=O)CH₂CH₃ | CH₃ | OCH₂CH₂CH₃ |
| A-477 | CH₂CH₃ | CH₂CH₃ | OCH₂CH₂CH₃ |
| A-478 | CF₃ | CH₂CH₃ | OCH₂CH₂CH₃ |
| A-479 | F | CH₂CH₃ | OCH₂CH₂CH₃ |
| A-480 | Cl | CH₂CH₃ | OCH₂CH₂CH₃ |
| A-481 | Br | CH₂CH₃ | OCH₂CH₂CH₃ |
| A-482 | I | CH₂CH₃ | OCH₂CH₂CH₃ |
| A-483 | OH | CH₂CH₃ | OCH₂CH₂CH₃ |
| A-484 | SH | CH₂CH₃ | OCH₂CH₂CH₃ |
| A-485 | SCH₃ | CH₂CH₃ | OCH₂CH₂CH₃ |
| A-486 | NH₂ | CH₂CH₃ | OCH₂CH₂CH₃ |
| A-487 | OCH₃ | CH₂CH₃ | OCH₂CH₂CH₃ |
| A-488 | OCHF₂ | CH₂CH₃ | OCH₂CH₂CH₃ |
| A-489 | OCHFCl | CH₂CH₃ | OCH₂CH₂CH₃ |
| A-490 | OCF₃ | CH₂CH₃ | OCH₂CH₂CH₃ |
| A-491 | OC(=O)CH₃ | CH₂CH₃ | OCH₂CH₂CH₃ |
| A-492 | OC(=O)CH₂CH₃ | CH₂CH₃ | OCH₂CH₂CH₃ |
| A-493 | CF₃ | CF₃ | OCH₂CH₂CH₃ |
| A-494 | F | CF₃ | OCH₂CH₂CH₃ |
| A-495 | Cl | CF₃ | OCH₂CH₂CH₃ |
| A-496 | Br | CF₃ | OCH₂CH₂CH₃ |
| A-497 | I | CF₃ | OCH₂CH₂CH₃ |
| A-498 | OH | CF₃ | OCH₂CH₂CH₃ |
| A-499 | SH | CF₃ | OCH₂CH₂CH₃ |
| A-500 | SCH₃ | CF₃ | OCH₂CH₂CH₃ |
| A-501 | NH₂ | CF₃ | OCH₂CH₂CH₃ |
| A-502 | OCH₃ | CF₃ | OCH₂CH₂CH₃ |
| A-503 | OCHF₂ | CF₃ | OCH₂CH₂CH₃ |
| A-504 | OCHFCl | CF₃ | OCH₂CH₂CH₃ |
| A-505 | OCF₃ | CF₃ | OCH₂CH₂CH₃ |
| A-506 | OC(=O)CH₃ | CF₃ | OCH₂CH₂CH₃ |
| A-507 | OC(=O)CH₂CH₃ | CF₃ | OCH₂CH₂CH₃ |
| A-508 | F | F | OCH₂CH₂CH₃ |
| A-509 | Cl | F | OCH₂CH₂CH₃ |
| A-510 | Br | F | OCH₂CH₂CH₃ |
| A-511 | I | F | OCH₂CH₂CH₃ |
| A-512 | OH | F | OCH₂CH₂CH₃ |
| A-513 | SH | F | OCH₂CH₂CH₃ |
| A-514 | SCH₃ | F | OCH₂CH₂CH₃ |
| A-515 | NH₂ | F | OCH₂CH₂CH₃ |
| A-516 | OCH₃ | F | OCH₂CH₂CH₃ |
| A-517 | OCHF₂ | F | OCH₂CH₂CH₃ |
| A-518 | OCHFCl | F | OCH₂CH₂CH₃ |
| A-519 | OCF₃ | F | OCH₂CH₂CH₃ |
| A-520 | OC(=O)CH₃ | F | OCH₂CH₂CH₃ |
| A-521 | OC(=O)CH₂CH₃ | F | OCH₂CH₂CH₃ |
| A-522 | Cl | Cl | OCH₂CH₂CH₃ |
| A-523 | Br | Cl | OCH₂CH₂CH₃ |
| A-524 | I | Cl | OCH₂CH₂CH₃ |
| A-525 | OH | Cl | OCH₂CH₂CH₃ |
| A-526 | SH | Cl | OCH₂CH₂CH₃ |
| A-527 | SCH₃ | Cl | OCH₂CH₂CH₃ |
| A-528 | NH₂ | Cl | OCH₂CH₂CH₃ |
| A-529 | OCH₃ | Cl | OCH₂CH₂CH₃ |
| A-530 | OCHF₂ | Cl | OCH₂CH₂CH₃ |
| A-531 | OCHFCl | Cl | OCH₂CH₂CH₃ |
| A-532 | OCF₃ | Cl | OCH₂CH₂CH₃ |
| A-533 | OC(=O)CH₃ | Cl | OCH₂CH₂CH₃ |
| A-534 | OC(=O)CH₂CH₃ | Cl | OCH₂CH₂CH₃ |
| A-535 | Br | Br | OCH₂CH₂CH₃ |
| A-536 | I | Br | OCH₂CH₂CH₃ |
| A-537 | OH | Br | OCH₂CH₂CH₃ |
| A-538 | SH | Br | OCH₂CH₂CH₃ |
| A-539 | SCH₃ | Br | OCH₂CH₂CH₃ |
| A-540 | NH₂ | Br | OCH₂CH₂CH₃ |
| A-541 | OCH₃ | Br | OCH₂CH₂CH₃ |
| A-542 | OCHF₂ | Br | OCH₂CH₂CH₃ |
| A-543 | OCHFCl | Br | OCH₂CH₂CH₃ |
| A-544 | OCF₃ | Br | OCH₂CH₂CH₃ |
| A-545 | OC(=O)CH₃ | Br | OCH₂CH₂CH₃ |
| A-546 | OC(=O)CH₂CH₃ | Br | OCH₂CH₂CH₃ |
| A-547 | I | I | OCH₂CH₂CH₃ |
| A-548 | OH | I | OCH₂CH₂CH₃ |
| A-549 | SH | I | OCH₂CH₂CH₃ |
| A-550 | SCH₃ | I | OCH₂CH₂CH₃ |
| A-551 | NH₂ | I | OCH₂CH₂CH₃ |
| A-552 | OCH₃ | I | OCH₂CH₂CH₃ |
| A-553 | OCHF₂ | I | OCH₂CH₂CH₃ |
| A-554 | OCHFCl | I | OCH₂CH₂CH₃ |
| A-555 | OCF₃ | I | OCH₂CH₂CH₃ |
| A-556 | OC(=O)CH₃ | I | OCH₂CH₂CH₃ |
| A-557 | OC(=O)CH₂CH₃ | I | OCH₂CH₂CH₃ |
| A-558 | OH | OH | OCH₂CH₂CH₃ |
| A-559 | SH | OH | OCH₂CH₂CH₃ |
| A-560 | SCH₃ | OH | OCH₂CH₂CH₃ |
| A-561 | NH₂ | OH | OCH₂CH₂CH₃ |
| A-562 | OCH₃ | OH | OCH₂CH₂CH₃ |
| A-563 | OCHF₂ | OH | OCH₂CH₂CH₃ |
| A-564 | OCHFCl | OH | OCH₂CH₂CH₃ |
| A-565 | OCF₃ | OH | OCH₂CH₂CH₃ |
| A-566 | OC(=O)CH₃ | OH | OCH₂CH₂CH₃ |
| A-567 | OC(=O)CH₂CH₃ | OH | OCH₂CH₂CH₃ |
| A-568 | SH | SH | OCH₂CH₂CH₃ |
| A-569 | SCH₃ | SH | OCH₂CH₂CH₃ |
| A-570 | NH₂ | SH | OCH₂CH₂CH₃ |
| A-571 | OCH₃ | SH | OCH₂CH₂CH₃ |
| A-572 | OCHF₂ | SH | OCH₂CH₂CH₃ |
| A-573 | OCHFCl | SH | OCH₂CH₂CH₃ |
| A-574 | OCF₃ | SH | OCH₂CH₂CH₃ |
| A-575 | OC(=O)CH₃ | SH | OCH₂CH₂CH₃ |
| A-576 | OC(=O)CH₂CH₃ | SH | OCH₂CH₂CH₃ |
| A-577 | SCH₃ | SCH₃ | OCH₂CH₂CH₃ |
| A-578 | NH₂ | SCH₃ | OCH₂CH₂CH₃ |
| A-579 | OCH₃ | SCH₃ | OCH₂CH₂CH₃ |
| A-580 | OCHF₂ | SCH₃ | OCH₂CH₂CH₃ |
| A-581 | OCHFCl | SCH₃ | OCH₂CH₂CH₃ |
| A-582 | OCF₃ | SCH₃ | OCH₂CH₂CH₃ |
| A-583 | OC(=O)CH₃ | SCH₃ | OCH₂CH₂CH₃ |
| A-584 | OC(=O)CH₂CH₃ | SCH₃ | OCH₂CH₂CH₃ |
| A-585 | NH₂ | NH₂ | OCH₂CH₂CH₃ |
| A-586 | OCH₃ | NH₂ | OCH₂CH₂CH₃ |
| A-587 | OCHF₂ | NH₂ | OCH₂CH₂CH₃ |
| A-588 | OCHFCl | NH₂ | OCH₂CH₂CH₃ |
| A-589 | OCF₃ | NH₂ | OCH₂CH₂CH₃ |
| A-590 | OC(=O)CH₃ | NH₂ | OCH₂CH₂CH₃ |
| A-591 | OC(=O)CH₂CH₃ | NH₂ | OCH₂CH₂CH₃ |
| A-592 | OCH₃ | OCH₃ | OCH₂CH₂CH₃ |
| A-593 | OCHF₂ | OCH₃ | OCH₂CH₂CH₃ |
| A-594 | OCHFCl | OCH₃ | OCH₂CH₂CH₃ |
| A-595 | OCF₃ | OCH₃ | OCH₂CH₂CH₃ |
| A-596 | OC(=O)CH₃ | OCH₃ | OCH₂CH₂CH₃ |
| A-597 | OC(=O)CH₂CH₃ | OCH₃ | OCH₂CH₂CH₃ |
| A-598 | OCHF₂ | OCHF₂ | OCH₂CH₂CH₃ |
| A-599 | OCHFCl | OCHF₂ | OCH₂CH₂CH₃ |
| A-600 | OCF₃ | OCHF₂ | OCH₂CH₂CH₃ |
| A-601 | OC(=O)CH₃ | OCHF₂ | OCH₂CH₂CH₃ |
| A-602 | OC(=O)CH₂CH₃ | OCHF₂ | OCH₂CH₂CH₃ |
| A-603 | OCHFCl | OCHFCl | OCH₂CH₂CH₃ |
| A-604 | OCF₃ | OCHFCl | OCH₂CH₂CH₃ |
| A-605 | OC(=O)CH₃ | OCHFCl | OCH₂CH₂CH₃ |
| A-606 | OC(=O)CH₂CH₃ | OCHFCl | OCH₂CH₂CH₃ |
| A-607 | OCF₃ | OCF₃ | OCH₂CH₂CH₃ |
| A-608 | OC(=O)CH₃ | OCF₃ | OCH₂CH₂CH₃ |
| A-609 | OC(=O)CH₂CH₃ | OCF₃ | OCH₂CH₂CH₃ |
| A-610 | OC(=O)CH₃ | OC(=O)CH₃ | OCH₂CH₂CH₃ |
| A-611 | OC(=O)CH₂CH₃ | OC(=O)CH₃ | OCH₂CH₂CH₃ |
| A-612 | OC(=O)CH₂CH₃ | OC(=O)CH₂CH₃ | OCH₂CH₂CH₃ |
| A-613 | CH₃ | CH₃ | OCH(CH₃)₂ |
| A-614 | CH₂CH₃ | CH₃ | OCH(CH₃)₂ |
| A-615 | CF₃ | CH₃ | OCH(CH₃)₂ |
| A-616 | F | CH₃ | OCH(CH₃)₂ |
| A-617 | Cl | CH₃ | OCH(CH₃)₂ |
| A-618 | Br | CH₃ | OCH(CH₃)₂ |
| A-619 | I | CH₃ | OCH(CH₃)₂ |
| A-620 | OH | CH₃ | OCH(CH₃)₂ |
| A-621 | SH | CH₃ | OCH(CH₃)₂ |
| A-622 | SCH₃ | CH₃ | OCH(CH₃)₂ |
| A-623 | NH₂ | CH₃ | OCH(CH₃)₂ |
| A-624 | OCH₃ | CH₃ | OCH(CH₃)₂ |
| A-625 | OCHF₂ | CH₃ | OCH(CH₃)₂ |
| A-626 | OCHFCl | CH₃ | OCH(CH₃)₂ |
| A-627 | OCF₃ | CH₃ | OCH(CH₃)₂ |
| A-628 | OC(=O)CH₃ | CH₃ | OCH(CH₃)₂ |
| A-629 | OC(=O)CH₂CH₃ | CH₃ | OCH(CH₃)₂ |
| A-630 | CH₂CH₃ | CH₂CH₃ | OCH(CH₃)₂ |
| A-631 | CF₃ | CH₂CH₃ | OCH(CH₃)₂ |
| A-632 | F | CH₂CH₃ | OCH(CH₃)₂ |
| A-633 | Cl | CH₂CH₃ | OCH(CH₃)₂ |
| A-634 | Br | CH₂CH₃ | OCH(CH₃)₂ |
| A-635 | I | CH₂CH₃ | OCH(CH₃)₂ |
| A-636 | OH | CH₂CH₃ | OCH(CH₃)₂ |
| A-637 | SH | CH₂CH₃ | OCH(CH₃)₂ |
| A-638 | SCH₃ | CH₂CH₃ | OCH(CH₃)₂ |
| A-639 | NH₂ | CH₂CH₃ | OCH(CH₃)₂ |
| A-640 | OCH₃ | CH₂CH₃ | OCH(CH₃)₂ |
| A-641 | OCHF₂ | CH₂CH₃ | OCH(CH₃)₂ |
| A-642 | OCHFCl | CH₂CH₃ | OCH(CH₃)₂ |
| A-643 | OCF₃ | CH₂CH₃ | OCH(CH₃)₂ |
| A-644 | OC(=O)CH₃ | CH₂CH₃ | OCH(CH₃)₂ |
| A-645 | OC(=O)CH₂CH₃ | CH₂CH₃ | OCH(CH₃)₂ |
| A-646 | CF₃ | CF₃ | OCH(CH₃)₂ |
| A-647 | F | CF₃ | OCH(CH₃)₂ |
| A-648 | Cl | CF₃ | OCH(CH₃)₂ |
| A-649 | Br | CF₃ | OCH(CH₃)₂ |
| A-650 | I | CF₃ | OCH(CH₃)₂ |
| A-651 | OH | CF₃ | OCH(CH₃)₂ |
| A-652 | SH | CF₃ | OCH(CH₃)₂ |
| A-653 | SCH₃ | CF₃ | OCH(CH₃)₂ |
| A-654 | NH₂ | CF₃ | OCH(CH₃)₂ |
| A-655 | OCH₃ | CF₃ | OCH(CH₃)₂ |
| A-656 | OCHF₂ | CF₃ | OCH(CH₃)₂ |
| A-657 | OCHFCl | CF₃ | OCH(CH₃)₂ |
| A-658 | OCF₃ | CF₃ | OCH(CH₃)₂ |
| A-659 | OC(=O)CH₃ | CF₃ | OCH(CH₃)₂ |
| A-660 | OC(=O)CH₂CH₃ | CF₃ | OCH(CH₃)₂ |
| A-661 | F | F | OCH(CH₃)₂ |
| A-662 | Cl | F | OCH(CH₃)₂ |
| A-663 | Br | F | OCH(CH₃)₂ |
| A-664 | I | F | OCH(CH₃)₂ |
| A-665 | OH | F | OCH(CH₃)₂ |
| A-666 | SH | F | OCH(CH₃)₂ |
| A-667 | SCH₃ | F | OCH(CH₃)₂ |
| A-668 | NH₂ | F | OCH(CH₃)₂ |
| A-669 | OCH₃ | F | OCH(CH₃)₂ |
| A-670 | OCHF₂ | F | OCH(CH₃)₂ |
| A-671 | OCHFCl | F | OCH(CH₃)₂ |
| A-672 | OCF₃ | F | OCH(CH₃)₂ |
| A-673 | OC(=O)CH₃ | F | OCH(CH₃)₂ |
| A-674 | OC(=O)CH₂CH₃ | F | OCH(CH₃)₂ |
| A-675 | Cl | Cl | OCH(CH₃)₂ |
| A-676 | Br | Cl | OCH(CH₃)₂ |
| A-677 | I | Cl | OCH(CH₃)₂ |
| A-678 | OH | Cl | OCH(CH₃)₂ |
| A-679 | SH | Cl | OCH(CH₃)₂ |
| A-680 | SCH₃ | Cl | OCH(CH₃)₂ |
| A-681 | NH₂ | Cl | OCH(CH₃)₂ |
| A-682 | OCH₃ | Cl | OCH(CH₃)₂ |
| A-683 | OCHF₂ | Cl | OCH(CH₃)₂ |
| A-684 | OCHFCl | Cl | OCH(CH₃)₂ |
| A-685 | OCF₃ | Cl | OCH(CH₃)₂ |
| A-686 | OC(=O)CH₃ | Cl | OCH(CH₃)₂ |
| A-687 | OC(=O)CH₂CH₃ | Cl | OCH(CH₃)₂ |
| A-688 | Br | Br | OCH(CH₃)₂ |
| A-689 | I | Br | OCH(CH₃)₂ |
| A-690 | OH | Br | OCH(CH₃)₂ |
| A-691 | SH | Br | OCH(CH₃)₂ |
| A-692 | SCH₃ | Br | OCH(CH₃)₂ |
| A-693 | NH₂ | Br | OCH(CH₃)₂ |
| A-694 | OCH₃ | Br | OCH(CH₃)₂ |
| A-695 | OCHF₂ | Br | OCH(CH₃)₂ |
| A-696 | OCHFCl | Br | OCH(CH₃)₂ |
| A-697 | OCF₃ | Br | OCH(CH₃)₂ |
| A-698 | OC(=O)CH₃ | Br | OCH(CH₃)₂ |
| A-699 | OC(=O)CH₂CH₃ | Br | OCH(CH₃)₂ |
| A-700 | I | I | OCH(CH₃)₂ |
| A-701 | OH | I | OCH(CH₃)₂ |
| A-702 | SH | I | OCH(CH₃)₂ |
| A-703 | SCH₃ | I | OCH(CH₃)₂ |
| A-704 | NH₂ | I | OCH(CH₃)₂ |
| A-705 | OCH₃ | I | OCH(CH₃)₂ |
| A-706 | OCHF₂ | I | OCH(CH₃)₂ |
| A-707 | OCHFCl | I | OCH(CH₃)₂ |
| A-708 | OCF₃ | I | OCH(CH₃)₂ |
| A-709 | OC(=O)CH₃ | I | OCH(CH₃)₂ |
| A-710 | OC(=O)CH₂CH₃ | I | OCH(CH₃)₂ |
| A-711 | OH | OH | OCH(CH₃)₂ |
| A-712 | SH | OH | OCH(CH₃)₂ |
| A-713 | SCH₃ | OH | OCH(CH₃)₂ |
| A-714 | NH₂ | OH | OCH(CH₃)₂ |
| A-715 | OCH₃ | OH | OCH(CH₃)₂ |
| A-716 | OCHF₂ | OH | OCH(CH₃)₂ |
| A-717 | OCHFCl | OH | OCH(CH₃)₂ |
| A-718 | OCF₃ | OH | OCH(CH₃)₂ |
| A-719 | OC(=O)CH₃ | OH | OCH(CH₃)₂ |
| A-720 | OC(=O)CH₂CH₃ | OH | OCH(CH₃)₂ |
| A-721 | SH | SH | OCH(CH₃)₂ |
| A-722 | SCH₃ | SH | OCH(CH₃)₂ |
| A-723 | NH₂ | SH | OCH(CH₃)₂ |
| A-724 | OCH₃ | SH | OCH(CH₃)₂ |
| A-725 | OCHF₂ | SH | OCH(CH₃)₂ |
| A-726 | OCHFCl | SH | OCH(CH₃)₂ |
| A-727 | OCF₃ | SH | OCH(CH₃)₂ |
| A-728 | OC(=O)CH₃ | SH | OCH(CH₃)₂ |
| A-729 | OC(=O)CH₂CH₃ | SH | OCH(CH₃)₂ |
| A-730 | SCH₃ | SCH₃ | OCH(CH₃)₂ |
| A-731 | NH₂ | SCH₃ | OCH(CH₃)₂ |
| A-732 | OCH₃ | SCH₃ | OCH(CH₃)₂ |
| A-733 | OCHF₂ | SCH₃ | OCH(CH₃)₂ |
| A-734 | OCHFCl | SCH₃ | OCH(CH₃)₂ |
| A-735 | OCF₃ | SCH₃ | OCH(CH₃)₂ |
| A-736 | OC(=O)CH₃ | SCH₃ | OCH(CH₃)₂ |
| A-737 | OC(=O)CH₂CH₃ | SCH₃ | OCH(CH₃)₂ |
| A-738 | NH₂ | NH₂ | OCH(CH₃)₂ |
| A-739 | OCH₃ | NH₂ | OCH(CH₃)₂ |
| A-740 | OCHF₂ | NH₂ | OCH(CH₃)₂ |
| A-741 | OCHFCl | NH₂ | OCH(CH₃)₂ |
| A-742 | OCF₃ | NH₂ | OCH(CH₃)₂ |
| A-743 | OC(=O)CH₃ | NH₂ | OCH(CH₃)₂ |
| A-744 | OC(=O)CH₂CH₃ | NH₂ | OCH(CH₃)₂ |
| A-745 | OCH₃ | OCH₃ | OCH(CH₃)₂ |
| A-746 | OCHF₂ | OCH₃ | OCH(CH₃)₂ |
| A-747 | OCHFCl | OCH₃ | OCH(CH₃)₂ |
| A-748 | OCF₃ | OCH₃ | OCH(CH₃)₂ |
| A-749 | OC(=O)CH₃ | OCH₃ | OCH(CH₃)₂ |
| A-750 | OC(=O)CH₂CH₃ | OCH₃ | OCH(CH₃)₂ |
| A-751 | OCHF₂ | OCHF₂ | OCH(CH₃)₂ |
| A-752 | OCHFCl | OCHF₂ | OCH(CH₃)₂ |
| A-753 | OCF₃ | OCHF₂ | OCH(CH₃)₂ |
| A-754 | OC(=O)CH₃ | OCHF₂ | OCH(CH₃)₂ |
| A-755 | OC(=O)CH₂CH₃ | OCHF₂ | OCH(CH₃)₂ |
| A-756 | OCHFCl | OCHFCl | OCH(CH₃)₂ |
| A-757 | OCF₃ | OCHFCl | OCH(CH₃)₂ |
| A-758 | OC(=O)CH₃ | OCHFCl | OCH(CH₃)₂ |
| A-759 | OC(=O)CH₂CH₃ | OCHFCl | OCH(CH₃)₂ |
| A-760 | OCF₃ | OCF₃ | OCH(CH₃)₂ |
| A-761 | OC(=O)CH₃ | OCF₃ | OCH(CH₃)₂ |
| A-762 | OC(=O)CH₂CH₃ | OCF₃ | OCH(CH₃)₂ |
| A-763 | OC(=O)CH₃ | OC(=O)CH₃ | OCH(CH₃)₂ |
| A-764 | OC(=O)CH₂CH₃ | OC(=O)CH₃ | OCH(CH₃)₂ |
| A-765 | OC(=O)CH₂CH₃ | OC(=O)CH₂CH₃ | OCH(CH₃)₂ |
| A-766 | CH₃ | CH₃ | OCH₂CH₂CH₂CH₃ |
| A-767 | CH₂CH₃ | CH₃ | OCH₂CH₂CH₂CH₃ |
| A-768 | CF₃ | CH₃ | OCH₂CH₂CH₂CH₃ |
| A-769 | F | CH₃ | OCH₂CH₂CH₂CH₃ |
| A-770 | Cl | CH₃ | OCH₂CH₂CH₂CH₃ |
| A-771 | Br | CH₃ | OCH₂CH₂CH₂CH₃ |
| A-772 | I | CH₃ | OCH₂CH₂CH₂CH₃ |
| A-773 | OH | CH₃ | OCH₂CH₂CH₂CH₃ |
| A-774 | SH | CH₃ | OCH₂CH₂CH₂CH₃ |
| A-775 | SCH₃ | CH₃ | OCH₂CH₂CH₂CH₃ |
| A-776 | NH₂ | CH₃ | OCH₂CH₂CH₂CH₃ |
| A-777 | OCH₃ | CH₃ | OCH₂CH₂CH₂CH₃ |
| A-778 | OCHF₂ | CH₃ | OCH₂CH₂CH₂CH₃ |
| A-779 | OCHFCl | CH₃ | OCH₂CH₂CH₂CH₃ |
| A-780 | OCF₃ | CH₃ | OCH₂CH₂CH₂CH₃ |
| A-781 | OC(=O)CH₃ | CH₃ | OCH₂CH₂CH₂CH₃ |
| A-782 | OC(=O)CH₂CH₃ | CH₃ | OCH₂CH₂CH₂CH₃ |
| A-783 | CH₂CH₃ | CH₂CH₃ | OCH₂CH₂CH₂CH₃ |
| A-784 | CF₃ | CH₂CH₃ | OCH₂CH₂CH₂CH₃ |
| A-785 | F | CH₂CH₃ | OCH₂CH₂CH₂CH₃ |
| A-786 | Cl | CH₂CH₃ | OCH₂CH₂CH₂CH₃ |
| A-787 | Br | CH₂CH₃ | OCH₂CH₂CH₂CH₃ |
| A-788 | I | CH₂CH₃ | OCH₂CH₂CH₂CH₃ |
| A-789 | OH | CH₂CH₃ | OCH₂CH₂CH₂CH₃ |
| A-790 | SH | CH₂CH₃ | OCH₂CH₂CH₂CH₃ |
| A-791 | SCH₃ | CH₂CH₃ | OCH₂CH₂CH₂CH₃ |
| A-792 | NH₂ | CH₂CH₃ | OCH₂CH₂CH₂CH₃ |
| A-793 | OCH₃ | CH₂CH₃ | OCH₂CH₂CH₂CH₃ |
| A-794 | OCHF₂ | CH₂CH₃ | OCH₂CH₂CH₂CH₃ |
| A-795 | OCHFCl | CH₂CH₃ | OCH₂CH₂CH₂CH₃ |
| A-796 | OCF₃ | CH₂CH₃ | OCH₂CH₂CH₂CH₃ |
| A-797 | OC(=O)CH₃ | CH₂CH₃ | OCH₂CH₂CH₂CH₃ |
| A-798 | OC(=O)CH₂CH₃ | CH₂CH₃ | OCH₂CH₂CH₂CH₃ |
| A-799 | CF₃ | CF₃ | OCH₂CH₂CH₂CH₃ |
| A-800 | F | CF₃ | OCH₂CH₂CH₂CH₃ |
| A-801 | Cl | CF₃ | OCH₂CH₂CH₂CH₃ |
| A-802 | Br | CF₃ | OCH₂CH₂CH₂CH₃ |
| A-803 | I | CF₃ | OCH₂CH₂CH₂CH₃ |
| A-804 | OH | CF₃ | OCH₂CH₂CH₂CH₃ |
| A-805 | SH | CF₃ | OCH₂CH₂CH₂CH₃ |
| A-806 | SCH₃ | CF₃ | OCH₂CH₂CH₂CH₃ |
| A-807 | NH₂ | CF₃ | OCH₂CH₂CH₂CH₃ |
| A-808 | OCH₃ | CF₃ | OCH₂CH₂CH₂CH₃ |
| A-809 | OCHF₂ | CF₃ | OCH₂CH₂CH₂CH₃ |
| A-810 | OCHFCl | CF₃ | OCH₂CH₂CH₂CH₃ |
| A-811 | OCF₃ | CF₃ | OCH₂CH₂CH₂CH₃ |
| A-812 | OC(=O)CH₃ | CF₃ | OCH₂CH₂CH₂CH₃ |
| A-813 | OC(=O)CH₂CH₃ | CF₃ | OCH₂CH₂CH₂CH₃ |
| A-814 | F | F | OCH₂CH₂CH₂CH₃ |
| A-815 | Cl | F | OCH₂CH₂CH₂CH₃ |
| A-816 | Br | F | OCH₂CH₂CH₂CH₃ |
| A-817 | I | F | OCH₂CH₂CH₂CH₃ |
| A-818 | OH | F | OCH₂CH₂CH₂CH₃ |
| A-819 | SH | F | OCH₂CH₂CH₂CH₃ |
| A-820 | SCH₃ | F | OCH₂CH₂CH₂CH₃ |
| A-821 | NH₂ | F | OCH₂CH₂CH₂CH₃ |
| A-822 | OCH₃ | F | OCH₂CH₂CH₂CH₃ |
| A-823 | OCHF₂ | F | OCH₂CH₂CH₂CH₃ |
| A-824 | OCHFCl | F | OCH₂CH₂CH₂CH₃ |
| A-825 | OCF₃ | F | OCH₂CH₂CH₂CH₃ |
| A-826 | OC(=O)CH₃ | F | OCH₂CH₂CH₂CH₃ |
| A-827 | OC(=O)CH₂CH₃ | F | OCH₂CH₂CH₂CH₃ |
| A-828 | Cl | Cl | OCH₂CH₂CH₂CH₃ |
| A-829 | Br | Cl | OCH₂CH₂CH₂CH₃ |
| A-830 | I | Cl | OCH₂CH₂CH₂CH₃ |
| A-831 | OH | Cl | OCH₂CH₂CH₂CH₃ |
| A-832 | SH | Cl | OCH₂CH₂CH₂CH₃ |
| A-833 | SCH₃ | Cl | OCH₂CH₂CH₂CH₃ |
| A-834 | NH₂ | Cl | OCH₂CH₂CH₂CH₃ |
| A-835 | OCH₃ | Cl | OCH₂CH₂CH₂CH₃ |
| A-836 | OCHF₂ | Cl | OCH₂CH₂CH₂CH₃ |
| A-837 | OCHFCl | Cl | OCH₂CH₂CH₂CH₃ |
| A-838 | OCF₃ | Cl | OCH₂CH₂CH₂CH₃ |
| A-839 | OC(=O)CH₃ | Cl | OCH₂CH₂CH₂CH₃ |
| A-840 | OC(=O)CH₂CH₃ | Cl | OCH₂CH₂CH₂CH₃ |
| A-841 | Br | Br | OCH₂CH₂CH₂CH₃ |
| A-842 | I | Br | OCH₂CH₂CH₂CH₃ |
| A-843 | OH | Br | OCH₂CH₂CH₂CH₃ |
| A-844 | SH | Br | OCH₂CH₂CH₂CH₃ |
| A-845 | SCH₃ | Br | OCH₂CH₂CH₂CH₃ |
| A-846 | NH₂ | Br | OCH₂CH₂CH₂CH₃ |
| A-847 | OCH₃ | Br | OCH₂CH₂CH₂CH₃ |
| A-848 | OCHF₂ | Br | OCH₂CH₂CH₂CH₃ |
| A-849 | OCHFCl | Br | OCH₂CH₂CH₂CH₃ |
| A-850 | OCF₃ | Br | OCH₂CH₂CH₂CH₃ |
| A-851 | OC(=O)CH₃ | Br | OCH₂CH₂CH₂CH₃ |
| A-852 | OC(=O)CH₂CH₃ | Br | OCH₂CH₂CH₂CH₃ |
| A-853 | I | I | OCH₂CH₂CH₂CH₃ |
| A-854 | OH | I | OCH₂CH₂CH₂CH₃ |
| A-855 | SH | I | OCH₂CH₂CH₂CH₃ |
| A-856 | SCH₃ | I | OCH₂CH₂CH₂CH₃ |
| A-857 | NH₂ | I | OCH₂CH₂CH₂CH₃ |
| A-858 | OCH₃ | I | OCH₂CH₂CH₂CH₃ |
| A-859 | OCHF₂ | I | OCH₂CH₂CH₂CH₃ |
| A-860 | OCHFCl | I | OCH₂CH₂CH₂CH₃ |
| A-861 | OCF₃ | I | OCH₂CH₂CH₂CH₃ |
| A-862 | OC(=O)CH₃ | I | OCH₂CH₂CH₂CH₃ |
| A-863 | OC(=O)CH₂CH₃ | I | OCH₂CH₂CH₂CH₃ |
| A-864 | OH | OH | OCH₂CH₂CH₂CH₃ |
| A-865 | SH | OH | OCH₂CH₂CH₂CH₃ |
| A-866 | SCH₃ | OH | OCH₂CH₂CH₂CH₃ |
| A-867 | NH₂ | OH | OCH₂CH₂CH₂CH₃ |
| A-868 | OCH₃ | OH | OCH₂CH₂CH₂CH₃ |
| A-869 | OCHF₂ | OH | OCH₂CH₂CH₂CH₃ |
| A-870 | OCHFCl | OH | OCH₂CH₂CH₂CH₃ |
| A-871 | OCF₃ | OH | OCH₂CH₂CH₂CH₃ |
| A-872 | OC(=O)CH₃ | OH | OCH₂CH₂CH₂CH₃ |
| A-873 | OC(=O)CH₂CH₃ | OH | OCH₂CH₂CH₂CH₃ |
| A-874 | SH | SH | OCH₂CH₂CH₂CH₃ |
| A-875 | SCH₃ | SH | OCH₂CH₂CH₂CH₃ |
| A-876 | NH₂ | SH | OCH₂CH₂CH₂CH₃ |
| A-877 | OCH₃ | SH | OCH₂CH₂CH₂CH₃ |
| A-878 | OCHF₂ | SH | OCH₂CH₂CH₂CH₃ |
| A-879 | OCHFCl | SH | OCH₂CH₂CH₂CH₃ |
| A-880 | OCF₃ | SH | OCH₂CH₂CH₂CH₃ |
| A-881 | OC(=O)CH₃ | SH | OCH₂CH₂CH₂CH₃ |
| A-882 | OC(=O)CH₂CH₃ | SH | OCH₂CH₂CH₂CH₃ |
| A-883 | SCH₃ | SCH₃ | OCH₂CH₂CH₂CH₃ |
| A-884 | NH₂ | SCH₃ | OCH₂CH₂CH₂CH₃ |
| A-885 | OCH₃ | SCH₃ | OCH₂CH₂CH₂CH₃ |
| A-886 | OCHF₂ | SCH₃ | OCH₂CH₂CH₂CH₃ |
| A-887 | OCHFCl | SCH₃ | OCH₂CH₂CH₂CH₃ |
| A-888 | OCF₃ | SCH₃ | OCH₂CH₂CH₂CH₃ |
| A-889 | OC(=O)CH₃ | SCH₃ | OCH₂CH₂CH₂CH₃ |
| A-890 | OC(=O)CH₂CH₃ | SCH₃ | OCH₂CH₂CH₂CH₃ |
| A-891 | NH₂ | NH₂ | OCH₂CH₂CH₂CH₃ |
| A-892 | OCH₃ | NH₂ | OCH₂CH₂CH₂CH₃ |
| A-893 | OCHF₂ | NH₂ | OCH₂CH₂CH₂CH₃ |
| A-894 | OCHFCl | NH₂ | OCH₂CH₂CH₂CH₃ |
| A-895 | OCF₃ | NH₂ | OCH₂CH₂CH₂CH₃ |
| A-896 | OC(=O)CH₃ | NH₂ | OCH₂CH₂CH₂CH₃ |
| A-897 | OC(=O)CH₂CH₃ | NH₂ | OCH₂CH₂CH₂CH₃ |
| A-898 | OCH₃ | OCH₃ | OCH₂CH₂CH₂CH₃ |
| A-899 | OCHF₂ | OCH₃ | OCH₂CH₂CH₂CH₃ |
| A-900 | OCHFCl | OCH₃ | OCH₂CH₂CH₂CH₃ |
| A-901 | OCF₃ | OCH₃ | OCH₂CH₂CH₂CH₃ |
| A-902 | OC(=O)CH₃ | OCH₃ | OCH₂CH₂CH₂CH₃ |
| A-903 | OC(=O)CH₂CH₃ | OCH₃ | OCH₂CH₂CH₂CH₃ |
| A-904 | OCHF₂ | OCHF₂ | OCH₂CH₂CH₂CH₃ |
| A-905 | OCHFCl | OCHF₂ | OCH₂CH₂CH₂CH₃ |
| A-906 | OCF₃ | OCHF₂ | OCH₂CH₂CH₂CH₃ |
| A-907 | OC(=O)CH₃ | OCHF₂ | OCH₂CH₂CH₂CH₃ |
| A-908 | OC(=O)CH₂CH₃ | OCHF₂ | OCH₂CH₂CH₂CH₃ |
| A-909 | OCHFCl | OCHFCl | OCH₂CH₂CH₂CH₃ |
| A-910 | OCF₃ | OCHFCl | OCH₂CH₂CH₂CH₃ |
| A-911 | OC(=O)CH₃ | OCHFCl | OCH₂CH₂CH₂CH₃ |
| A-912 | OC(=O)CH₂CH₃ | OCHFCl | OCH₂CH₂CH_{Z}CH₃ |
| A-913 | OCF₃ | OCF₃ | OCH₂CH₂CH₂CH₃ |
| A-914 | OC(=O)CH₃ | OCF₃ | OCH₂CH₂CH₂CH₃ |
| A-915 | OC(=O)CH₂CH₃ | OCF₃ | OCH₂CH₂CH₂CH₃ |
| A-916 | OC(=O)CH₃ | OC(=O)CH₃ | OCH₂CH₂CH₂CH₃ |
| A-917 | OC(=O)CH₂CH₃ | OC(=O)CH3 | OCH₂CH₂CH₂CH₃ |
| A-918 | OC(=O)CH₂CH₃ | OC (=O)CH₂CH₃ | OCH₂CH₂CH₂CH₃ |

Die Verbindungen der Formel I sind auf verschiedenen Wegen zugänglich. Beispielsweise erhält man Verbindungen der Formel I, in denen X Sauerstoff bedeutet, durch Umsetzung von Aldehyden der Formel II, 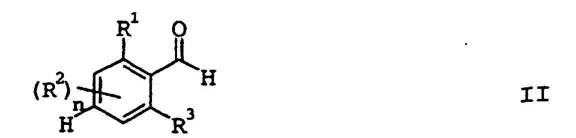 wobei die Variablen R¹, R², R³ und n die für Formel I angegebene Bedeutung haben und durch eine nach der Umsetzung abzuspaltende Schutzgruppe Q geschützt sein können, mit Halogenbenzolderivaten der Formel III, 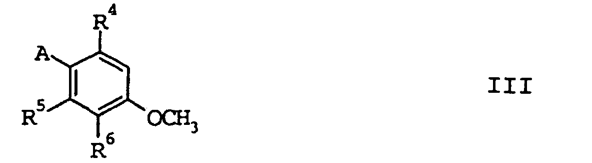 wobei die Variablen R⁴, R⁵ und R⁶ die für Formel I angegebene Bedeutung haben und A für Halogen steht, und A in einem Zwischenschritt in eine Gruppe B überführt wird, wobei B für ein geeignetes Metall oder eine geeignete Halogenmetallgruppe steht.

Als Gruppen A kommen Fluor, Chlor, Brom oder Iod, bevorzugt Chlor oder Brom in Betracht.

Geeignete Gruppen B sind beispielsweise Li, Na, MgY (Y = Halogen, insbesondere Br), Sn(R')₂ (R' = CH₃, CH₂CH₃), ZnR', Cu(CN)ZnI [vgl. J. March, Advanced Organic Chemistry, J. Wiley & Sons, New York, 1992, S. 920 - 929 und zit. Lit.];

Bevorzugte Gruppen B sind Na und MgY.

Die Umsetzung erfolgt durch Überführung der Halogengruppe A in die Gruppe B in an sich bekannter Weise und nachfolgender in situ Umsetzung der so erhaltenen Benzolderivate mit Aldehyden der Formel II [vgl. J. March, Advanced Organic Chemistry, J. Wiley & Sons, New York, 1992, S. 920 - 929 und zit. Lit.].

Wenn B Li, Na oder MgY bedeutet, erfolgt die Umsetzung beispielsweise bei Temperaturen von -20 °C bis 150 °C, vorzugsweise 20 °C bis 100 °C, in einem inerten organischen Lösungsmittel und mit mindestens äquimolaren Mengen Li, Na oder Mg bezogen auf das Halogenbenzol III, und, wenn B Li oder Na bedeutet, gegebenenfalls unter reaktionsbeschleunigenden Bedingungen wie der Zugabe von Dibromethan oder Iod oder dem Einsatz von Ultraschall [vgl. Lit. J. March, Advanced Organic Chemistry, J. Wiley & Sons, New York, 1992, S. 920 - 929 und zit. Lit.].

Die Einführung der Schutzgruppe Q in die Aldehyde der Formel II erfolgt gemäß literaturbekannter Methoden [vgl. T. W. Greene, Protective Groups in Organic Chemistry, J. Wiley & Sons, 1991, S. 10 - 142].

Als Schutzgruppen für R¹, R², R³ = Hydroxy, Mercapto, oder Amino kommen beispielsweise unter lewis-sauren Bedingungen abspaltbare Gruppen, wie Alkyl, insbesondere Methyl in Betracht.

In einer bevorzugten Ausgestaltung der Reaktion sind die Variablen R¹, R² und R³ unter den Reaktionsbedingungen inert.

Für die Darstellung von Verbindungen I, in denen R¹ und R³ Carbo-nylfunktionalitäten enthalten, ist das unten beschriebene Verfahren ausgehend von den entsprechenden Benzophenonen IV bevorzugt.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, bevorzugt Ether, ganz besonders bevorzugt Diethylether und Tetrahydrofuran.

Es können auch Gemische der genannten Lösungsmittel verwendet werden.

Die Edukte werden im allgemeinen in äquimolaren Mengen miteinander umgesetzt. Es kann für die Ausbeute vorteilhaft sein, Verbindungen der Formel III in einem Überschuß bezogen auf Verbindungen der Formel II einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen I benötigten Ausgangsstoffe der Formel II können beispielsweise durch Reduktion der entsprechenden Benzoesäuren oder Benzoesäureester mit Disiobutylaluminiumhydrid hergestellt werden, welche bespielsweise in EP-A 727 141 beschrieben sind, oder können auf an sich bekannte Weise hergestellt werden [vgl. J. March, Advanced Organic Chemistry, J. Wiley & Sons, New York, 1992, S. 501 - 521, S. 641 - 758, S. 982 - 1161.].

Die für die Herstellung der Verbindungen I benötigten Ausgangsstoffe der Formel III können gemäß literaturbekannten Methoden aus Gallussäure bzw. ihren Derivaten oder auf an sich bekannte Weise hergestellt werden [vgl. J. March, Advanced Organic Chemistry, J. Wiley & Sons, New York, 1992, S. 501 - 521, S. 641 - 758, S. 982 - 1161.].

Verbindungen der Formel I, in denen X Sauerstoff bedeutet, können auch durch Reduktion der analogen Benzophenonderivate der Formel IV, 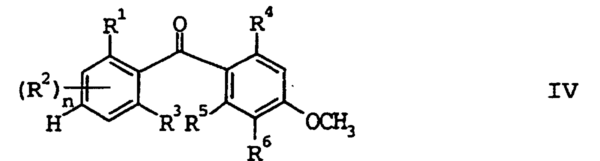 hergestellt werden, wobei die Variablen die für Formel I angegebene Bedeutung haben oder eine Gruppe bedeuten, die nach der Umsetzung dazu umgewandelt werden kann, mit Hydriden oder durch katalytische Reduktion mit H₂.

Als Hydride eignen sich vorzugsweise Bor- und Aluminiumhydride wie NaBH₄ oder LiAlH₄ [vgl. M. Hudlicky, Reductions in Organic Chemistry, ACS Monograph 188, American Chemical Society, Washington D.C., 1996, S. 152 ff; Tetrahedron Lett. 28 (4), S. 4725 - 4728 (1987)] oder elementares Aluminium in Ammoniak [vgl. Chem. Soc. Jpn. 63(1), S. 290 - 292 (1990)].

Die Umsetzung erfolgt üblicherweise bei Temperaturen von -78 °C bis 120 °C, vorzugsweise -78 °C bis 0 °C, in einem inerten organischen Lösungsmittel.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, bevorzugt Ether, besonders bevorzugt Tetrahydrofuran. Es können auch Gemische der genannten Lösungsmittel verwendet werden.

Die Edukte werden im allgemeinen in äquimolaren Mengen miteinander umgesetzt. Es kann für die Ausbeute vorteilhaft sein, das Reduktionsmittel in einem Überschuß bezogen auf Verbindungen der Formel IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Reduktion kann auch in an sich bekannter Weise mit H₂ und Katalysatoren auf der Basis von Übergangsmetallen durchgeführt , werden. Geeignete Übergangsmetalle sind Palladium, Platin, Rhodium und Ruthenium.

Diese Umsetzung erfolgt üblicherweise bei Temperaturen von 0°C bis 150°C, vorzugsweise 0°C bis 50°C, in einem inerten organischen Lösungsmittel [vgl. Organotransition Metal Chemistry, Academic Press, New York, 1974, S. 65-70 und zit. Lit.;
JP-A 10273455].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Alkohole wie Methanol, Ethanol, n-Propanol, Isopropanol, n-Butanol und tert.-Butanol, besonders bevorzugt Methanol, Ethanol oder Isopropanol. Es können auch Gemische der genannten Lösungsmittel verwendet werden.

In einer bevorzugten Ausgestaltung der Reduktion mit H₂ werden chirale Katalysatoren verwendet, so daß selektiv das (R)- oder (S)-Isomer des Benzhydrylalkohols I erhalten werden können [vgl. Org. Lett. 2(5), S. 659 - 662, (2000)].

Wenn die Variablen R¹, R² oder R³ unter den Reaktionsbedingungen reduzierbare Gruppen wie Nitro oder Formyl bedeuten, kann es notwendig sein, diese Gruppen nach der Reaktion durch selektive Oxidation zu reetablieren. Es kann auch vorteilhaft sein, solche reduzierbaren Gruppen erst nach erfolgter Reduktion der Benzophenone IV durch Oxidation entsprechender Gruppen einzuführen (beispielsweise eine Nitro-Gruppe durch Oxidation einer Amino-Gruppe oder eine Formyl-Gruppe durch Oxidation einer Hydroxy-Gruppe).

Beispielsweise können Verbindungen I, in denen R¹ Formyl bedeutet, durch selektive Oxidation von entsprechenden Verbindungen, in denen R¹ Hydroxymethyl ist, welches bei der erfindungsgemäßen Reduktion aus einer Formyl-Gruppe enstehen kann, erhalten werden [vgl. M. Hudlicky, Oxidations in Organic Chemistry, ACS Monograph 186, American Chemical Society, Washington D.C., 1990, S. 114-127].

Es kann auch vorteilhaft sein, Verbindungen der Formel I, in denen R¹ Hydroxy bedeutet, durch Reduktion von Verbindungen der Formel IV, in denen R¹ Alkylcarbonyloxy ist, zu erhalten. Dabei werden die die Phenylgruppen verbrückende Ketogruppe und R¹ gleichzeitig reduziert.

Für Verbindungen I, in denen die Variablen R¹, R², R³ oder R⁴ Alkenyl bzw. Alkinyl bedeuten, ist das oben beschriebene Verfahren ausgehend von Halogenbenzolderivaten III und Aldehyden II bevorzugt.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Verbindungen I benötigten Ausgangsstoffe der Formel IV sind in der Literatur bekannt (beispielsweise aus EP-A 727 141) oder können gemäß der zitierten Literatur hergestellt werden.

Sofern einzelne Verbindungen I nicht auf den voranstehend beschriebenen Wegen zugänglich sind, können sie durch Derivatisierung anderer Verbindungen I hergestellt werden.

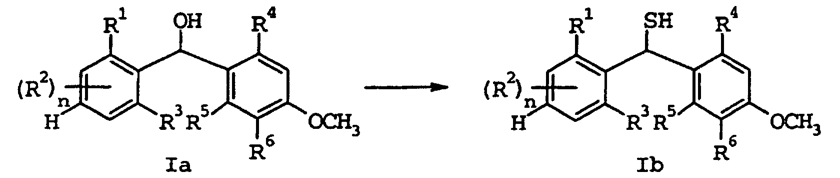

Die Benzhydrylthiole Ib können ausgehend von den entsprechenden Alkoholen Ia unter literaturbekannten Bedingungen erhalten werden. Die Umsetzung erfolgt üblicherweise bei Temperaturen von 0°C bis 180°C, vorzugsweise 20°C bis 140°C in einem inerten organischen Lösungsmittel [vgl. Liebigs Ann. Chem., S. 177 (1989)].

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, und Dimethylenglykol, besonders bevorzugt Toluol und Dimethylenglykol.

Es können auch Gemische der genannten Lösungsmittel verwendet werden.

Als Schwefelungsagentien kommen beispielsweise Phosphorpentasulfid oder das Lawesson-Reagenz in Betracht.

Die Edukte werden im allgemeinen in äquimolaren Mengen miteinander umgesetzt. Es kann für die Ausbeute vorteilhaft sein, das Schwefelungsreagenz in einem Überschuß bezogen auf Ia einzusetzen.

Benzhydrylthiole Ib lassen sich auch durch Reduktion der entsprechenden Thioketone V erhalten, welche aus den Benzophenonen IV hergestellt werden können.

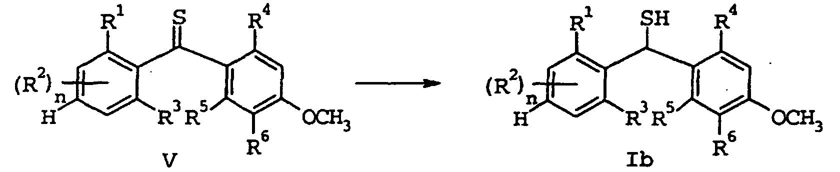

Die Reduktion zu den Benzhydrylthiolen Ib kann beispielsweise mit mit Bor- oder Aluminiumhydriden erfolgen [vgl. Synth. Commun. 23 (9), S. 1267 - 1271 (1993); Tetrahedron: Asymmetry 7(12), S. 3553 - 3558 (1996); Can. J. Chem. 48, S. 3593, (1970).] oder mit elementarem Ytterbium in Tetrahydrofuran [vgl. Chem. Lett. 3, S. 611 (1994)].

Die Umsetzung der Benzophenone IV zu den Thioketonen V erfolgt analog zur oben beschriebenen Umsetzung der Benzhydrylalkohole Ia.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten*, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung forgender Pfranzenkrankheiten:
- Alternaria-Arten an Gemüse und Obst,
- Botrytis cinerea (Grauschimmel) an Erdbeeren, Gemüse, Zier- , pflanzen und Reben,
- Cercospora arachidicola an Erdnüssen,
- Erysiphe cichoracearum und Sphaerotheca fuliginea an Kürbisgewächsen,
- Erysiphe graminis (echter Mehltau) an Getreide,
- Fusarium- und Verticillium-Arten an verschiedenen Pflanzen,
- Helminthosporium-Arten an Getreide,
- Mycosphaerella-Arten an Bananen und Erdnüssen,
- Phytophthora infestans an Kartoffeln und Tomaten,
- Plasmopara viticola an Reben,
- Podosphaera leucotricha an Äpfeln,
- Pseudocercosporella herpotrichoides an Weizen und Gerste,
- Pseudoperonospora-Arten an Hopfen und Gurken,
- Puccinia-Arten an Getreide,
- Pyricularia oryzae an Reis,
- Rhizoctonia-Arten an Baumwolle, Reis und Rasen,
- Septoria nodorum an Weizen,
- Uncinula necator an Reben,
- Ustilago-Arten an Getreide und Zuckerrohr, sowie
- Venturia-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im , Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt.
   Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in 5 einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylenbis-(thiocarbamoyl)disulfid;
- Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2,4-Dichlor-6-(o-chloranilino)-s-triazin, 0,0-Diethylphthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-ylethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, , (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yloxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid,
- sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl-ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid; 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, Z-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel I

Herstellung von 5-Brom-6,6'-dimethyl-2,2',3',4'-tetramethoxybenzhydrylalkohol 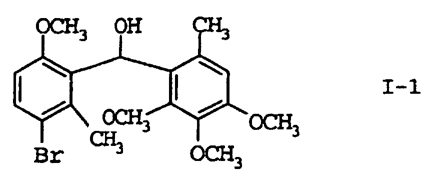 2,36 g 2,3,4-Trimethoxy-6-methyl-brombenzol und 0,32 g Magnesiumspäne wurden in 5 ml wasserfreiem Tetrahydrofuran vorgelegt, mit 0,57 ml 1,2-Dibromethan versetzt und etwa 40 Min. auf Rückflußtemperatur erhitzt. Nach Abkühlen auf 30°C wurden 1,40 g 5-Brom-2-methoxy-6-methyl-benzaldehyd zugetropft und die Lösung etwa zwei Stunden gerührt. Nach Zugabe von Wasser/2N Salzsäure (1/1), Extraktion der wässerigen Phase mit Essigester, Waschen der organischen Phasen mit Wasser, Trocknung und Abdestillieren des Lösungsmittels wurde der Rückstand säulenchromatographisch gereinigt (Eluent Petrolether/Essigsäureethylester 85/5). Es wurden 1,5 g der Titelverbindung als violettes, zähflüssiges Öl isoliert.

### Beispiel 2

Herstellung von 2,5-Dichlor-6,6'-dimethyl-2',3',4'-trimethoxybenzhydrylalkohol 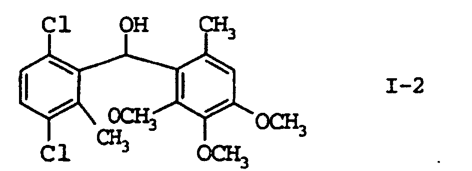 Unter Schutzgasbedingungen wurde bei etwa -78°C zu einer Lösung von 0,37 g 2,5-Dichlor-6,6'-dimethyl-2',3',4'-trimethoxy-benzophenon in 20 ml wasserfreiem Tetrahydrofuran 1 ml einer 1-molaren Lösung von LiAlH₄ in Tetrahydrofuran zugetropft und etwa 1 Stunde gerührt. Nach Versetzen der Reaktionslösung mit ges. NaHCO₃-Lösung bei 20-25°C, Extraktion der Lösung mit Essigsäureethylester, Waschen der organische Phase mit Wasser und ges. NaCl-Lösung sowie Trocknung wurde das Lösungsmittel abdestilliert. Aus dem Rückstand erhielt man nach säulenchromatographischer Reinigung (Eluent: Essigsäureethylester/Cyclohexan 1:9) 150 mg der Titelverbindung als gelbes, zähflüssiges Öl.

### Beispiel 3

Herstellung von 5-Chlor-2-hydroxy-6,6'-dimethyl-2',3',4'-trimethoxy-benzhydrylalkohol 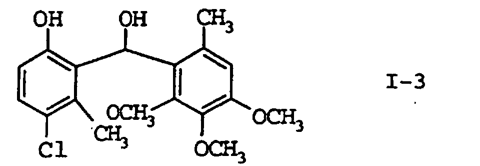 Zu einer Lösung von 0.86 g 5-Chlor-2-hydroxy-6,6'dimethyl-2',3',4'-trimethoxy-benzophenon in 10 ml wasserfreiem Tetrahydrofuran (THF) wurden bei -20°C unter Schutzgasatmosphäre 3 mmol einer 1-molaren Lösung von LiAlH₄ in THF zugetropft. Nach Erwärmen auf Raumtemperatur wurde die Reaktionsmischung mit Eiswasser versetzt, mit verdünnter Salzsäure angesäuert und mit Essigsäureethylester extrahiert. Die vereinten organischen Phasen wurden mit ges. NaCl-Lösung gewaschen, getrocknet und säulenchromatographisch gereinigt (Eluent: Essigsäureethylester/Cyclohexan 3:7). Es wurden 0.46 mg der Titelverbindung als weißer Feststoff vom Fp. 140-142°C isoliert.

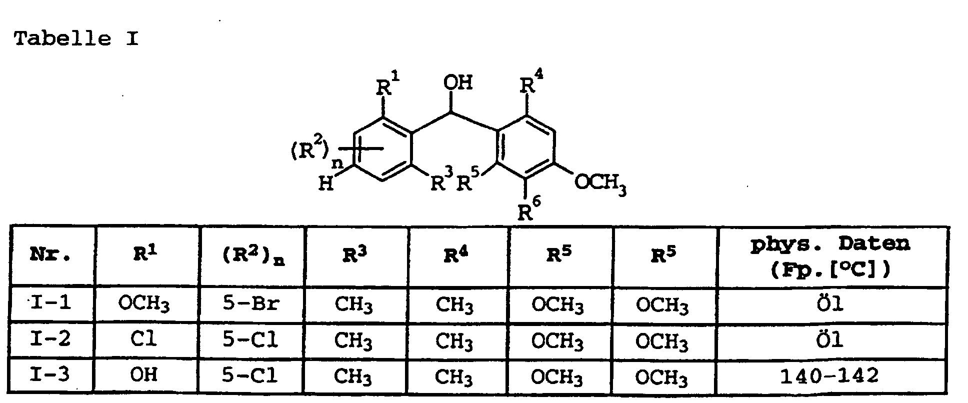

Beispiele für die Wirkung gegen Schadpilze

Die fungizide Wirkung der Verbindungen der allgemeinen Formel I ließ sich durch die folgende Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

Nach Abschluß der Versuche wurde die jeweils niedrigste Konzentration ermittelt, bei der die Verbindungen im Vergleich zu unbehandelten Kontrollen noch eine 80 bis 100%-ige Hemmung hervorriefen (Wirkschwelle bzw. Minimalkonzentration).

### Anwendungsbeispiel 1 - Wirksamkeit gegen Weizenmehltau

Blätter von in Töpfen gewachsenen Weizenkeimlingen der Sorte "Kanzler" wurden mit wäßriger Wirkstoffaufbereitung, die aus einer Stammlösung bestehend aus 10 % Wirkstoff, 63 % Cyclohexanon und 27 % Emulgiermittel angesetzt wurde, bis zur Tropfnässe besprüht und 24 Stunden nach dem Antrocknen des Spritzbelages mit Sporen des Weizenmehltaus (Erysiphe graminis forma specialis tritici) bestäubt. Die Versuchspflanzen wurden anschließend im Gewächshaus bei Temperaturen zwischen 20 und 24°C und 60 bis 90 % rel. Luftfeuchtigkeit aufgestellt. Nach 7 Tagen wurde das Ausmaß der Mehltauentwicklung visuell in % Befall der gesamten Blattfläche ermittelt.

In diesem Test zeigten die mit 4 bzw. 16 ppm der Wirkstoffe I-1, I-2 und I-3 behandelten Pflanzen einen Befall von jeweils höchstens 30 %, während die unbehandelten Pflanzen zu 90 % befallen waren.

## Patentansprüche

1. Benzhydrylderivate der Formel I, 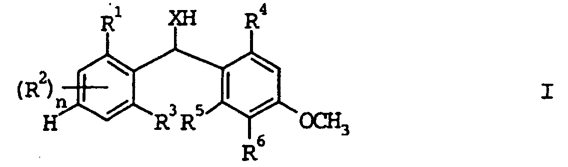 wobei der Index und die Variablen die folgende Bedeutung haben:
X Sauerstoff oder Schwefel;
R¹,R³ Halogen, Cyano, Nitro, Hydroxy, Mercapto, Amino, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, C₁-C₆-Alkylcarbonyloxy, Formyloxy, C₁-C₆-Alkylthio, C₂-C₆-Alkenylthio, C₂-C₆-Alkinylthio, C₁-C₆-Alkylamino, Di- C₁-C₆-alkylamino, C₁-C₆-Alkylcarbonyl oder Formyl, wobei die Kohlenstoffatome in den genannten Resten partiell oder vollständig halogeniert sein können;
R² Halogen, Cyano, Nitro, Hydroxy, Mercapto, Amino, C₁-C₆-Alkyl, C₁-C₆-Alkoxy, C₁-C₆-Haloalkyl oder C₁-C₆-Haloalkoxy, wobei die Gruppen R² verschieden sein können, wenn n=2 ist;
R⁴ C₁-C₆-Alkyl, C₂-C₆-Alkenyl oder C₂-C₆-Alkinyl, wobei die Kohlenstoffatome in diesen Resten unsubstituiert oder teilweise oder vollständig halogeniert sein können;
R⁵,R⁶ Hydroxy, C₁-C₆-Alkyl, C₂-C₆-Alkenyl, C₁-C₆-Haloalkyl, C₂-C₆-Haloalkenyl, C₁-C₆-Alkoxy, C₂-C₆-Alkenyloxy, C₁-C₆-Haloalkoxy, C₂-C₆-Haloalkenyloxy, C₃-C₈-Cycloalkyl, C₃-C₈-Cycloalkyl-C₁-C₃-alkyl, C₃-C₈-Cycloalkoxy oder C₃-C₈-Cycloalkyl-C₁-C₃-alkoxy;
n 0, 1 oder 2;

2. Verbindungen der Formel I gemäß Anspruch 1, wobei X Sauerstoff bedeutet.

3. Verbindungen der Formel I gemäß den Ansprüchen 1 oder 2, wobei die Variablen die folgende Bedeutung haben:
R¹,R³ unabhängig voneinander Halogen, Hydroxy, Amino, Mercapto, Nitro, Cyano, C₁-C₆-Alkyl, C₁-C₆-Haloalkyl, C₁-C₆-Alkoxy, C₁-C₆-Haloalkoxy, C₁-C₆-Alkylcarbonyloxy, C₁-C₆-Haloalkylcarbonyloxy, C₁-C₆-Alkylthio, C₁-C₆-Haloalkylthio, C₁-C₆-Alkylcarbonyl oder C₁-C₆-Haloalkylcarbonyl;

4. Verbindungen der Formel I gemäß den Ansprüchen 1 bis 3, wobei
R² Halogen, Hydroxy, C₁-C₆-Alkyl, C₁-C₆-Haloalkyl, C₁-C₆-Alkoxy oder C₁-C₆-Haloalkoxy bedeutet.

5. Verbindungen der Formel I gemäß den Ansprüchen 1 bis 4, wobei
R⁴ C₁-C₆-Alkyl oder C₁-C₆-Haloalkyl
bedeutet.

6. Verbindungen der Formel I gemäß den Ansprüchen 1 bis 5, wobei
R⁵,R⁶ Hydroxy, C₁-C₆-Alkoxy, C₂-C₆-Alkenyloxy, C₁-C₆-Haloalkoxy oder C₂-C₆-Haloalkenyloxy
bedeuten.

7. Verbindungen der Formel I gemäß den Ansprüchen 1 oder 2, wobei die Variablen die folgende Bedeutung haben:
R¹,R³ Halogen, Hydroxy, Amino, Mercapto, C₁-C₆-Alkyl, C₁-C₆-Haloalkyl, C₁-C₆-Alkoxy, C₁-C₆-Haloalkoxy oder C₁-C₆-Alkylcarbonyl;
R² Halogen, Hydroxy, C₁-C₆-Alkyl, C₁-C₆-Haloalkyl, C₁-C₆-Alkoxy oder C₁-C₆-Haloalkoxy;
R⁴ Methyl;
R⁵,R⁶ C₁-C₆ Alkoxy;
n 0 oder 1.

8. Verfahren zur Herstellung von Verbindungen der Formel I gemäß Anspruch 1, in denen X Sauerstoff bedeutet, durch Umsetzung von Aldehyden der Formel II, 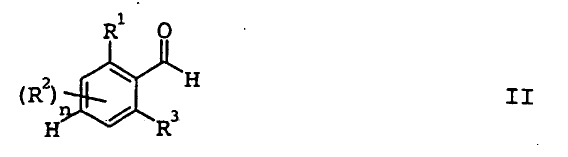 wobei die Variablen R¹, R², R³ und n die für Formel I angegebene Bedeutung haben und durch eine nach der Umsetzung abzuspaltende Schutzgruppe Q geschützt sein können, mit Halogenbenzolderivaten der Formel III, 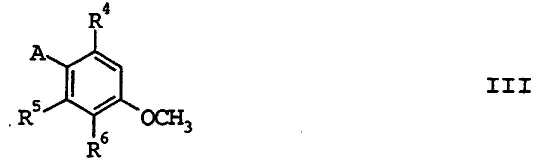 wobei die Variablen R⁴, R⁵ und R⁶ die für Formel I angegebene Bedeutung haben und A für Halogen steht, und A in einem Zwischenschritt in eine Gruppe B überführt wird, wobei B für ein geeignetes Metall oder eine geeignete Halogenmetallgruppe steht.

9. Verfahren zur Herstellung von Verbindungen der Formel I gemäß Anspruch 1, in der X Sauerstoff bedeutet, durch Reduktion von Benzophenonderivaten der Formel IV, 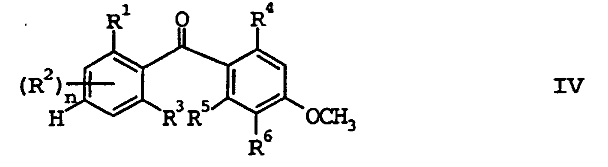 wobei die Variablen die für Formel I angegebene Bedeutung haben oder eine Gruppe bedeuten, die nach der Umsetzung dazu umgewandelt werden kann, mit Hydriden oder durch katalytische Reduktion mit H₂.

10. Zur Bekämpfung von pflanzenpathogenen Schadpilzen geeignetes Mittel, enthaltend einen festen oder flüssigen Trägerstoff und eine Verbindung der allgemeinen Formel I gemäß Anspruch 1.

11. Verwendung der Verbindungen I gemäß Anspruch 1 zur Herstellung eines zur Bekämpfung von pflanzenpathogenen Schadpilzen geeigneten Mittels.

12. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Verbindung der allgemeinen Formel I gemäß Anspruch 1 behandelt.

## Claims

1. A benzhydryl derivative of the formula I, 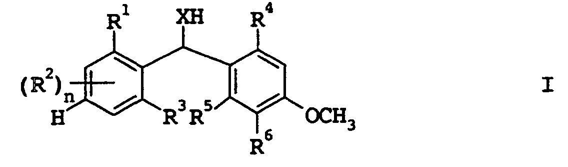 where the index and the variables are as defined below:
X is oxygen or sulfur;
R¹,R³ are halogen, cyano, nitro, hydroxyl, mercapto, amino, C₁-C₆-alkyl, C₂-C₆-alkenyl, C₂-C₆-alkynyl, C₁-C₆-alkoxy, C₂-C₆-alkenyloxy, C₂-C₆-alkynyloxy, C₁-C₆-alkylcarbonyloxy, formyloxy, C₁-C₆-alkylthio, C₂-C₆-alkenylthio, C₂-C₆-alkynylthio, C₁-C₆-alkylamino, di-C₁-C₆-alkylamino, C₁-C₆-alkylcarbonyl or formyl, where the carbon atoms in the radicals mentioned may be partially or fully halogenated;
R² is halogen, cyano, nitro, hydroxyl, mercapto, amino, C₁-C₆-alkyl, C₁-C₆-alkoxy, C₁-C₆-haloalkyl or C₁-C₆-haloalkoxy, where the groups R² may be different if n=2;
R⁴ is C₁-C₆-alkyl, C₂-C₆-alkenyl or C₂-C₆-alkynyl, where the carbon atoms in these radicals may be unsubstituted or partially or fully halogenated;
R⁵,R⁶ are hydroxyl, C₁-C₆-alkyl, C₂-C₆-alkenyl, C₁-C₆-haloalkyl, C₂-C₆-haloalkenyl, C₁-C₆-alkoxy, C₂-C₆-alkenyloxy, C₁-C₆-haloalkoxy, C₂-C₆-haloalkenyloxy, C₃-C₈-cycloalkyl, C₃-C₈-cycloalkyl-C₁-C₃-alkyl, C₃-C₈-cycloalkoxy or C₃-C₈-cycloalkyl-C₁-C₃-alkoxy;
n is 0, 1 or 2.

2. A compound of the formula I as claimed in claim 1 where X is oxygen.

3. A compound of the formula I as claimed in claim 1 or 2 where the variables are as defined below:
R¹,R³ independently of one another are halogen, hydroxyl, amino, mercapto, nitro, cyano, C₁-C₆-alkyl, C₁-C₆-haloalkyl, C₁-C₆-alkoxy, C₁-C₆-haloalkoxy, C₁-C₆-alkylcarbonyloxy, C₁-C₆-haloalkylcarbonyloxy, C₁-C₆-alkylthio, C₁-C₆-haloalkylthio, C₁-C₆-alkylcarbonyl or C₁-C₆-haloalkylcarbonyl.

4. A compound of the formula I as claimed in any of claims 1 to 3 where
R² is halogen, hydroxyl, C₁-C₆-alkyl, C₁-C₆-haloalkyl, C₁-C₆-alkoxy or C₁-C₆-haloalkoxy.

5. A compound of the formula I as claimed in any of claims 1 to 4 where
R⁴ is C₁-C₆-alkyl or C₁-C₆-haloalkyl.

6. A compound of the formula I as claimed in any of claims 1 to 5 where
R⁵,R⁶ are hydroxyl, C₁-C₆-alkoxy, C₂-C₆-alkenyloxy, C₁-C₆-haloalkoxy or C₂-C₆-haloalkenyloxy.

7. A compound of the formula I as claimed in claim 1 or 2, where the variables are as defined below:
R¹,R³ are halogen, hydroxyl, amino, mercapto, C₁-C₆-alkyl, C₁-C₆-haloalkyl, C₁-C₆-alkoxy, C₁-C₆-haloalkoxy or C₁-C₆-alkylcarbonyl;
R² is halogen, hydroxyl, C₁-C₆-alkyl, C₁-C₆-haloalkyl, C₁-C₆-alkoxy or C₁-C₆-haloalkoxy;
R⁴ is methyl;
R⁵,R⁶ are C₁-C₆-alkoxy;
n is 0 or 1.

8. A process for preparing compounds of the formula I as claimed in claim 1 in which X is oxygen, by reacting aldehydes of the formula II, 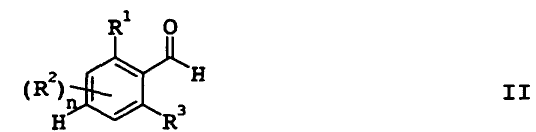 where the variables R¹, R², R³ and n are as defined for formula I and may be protected by a protective group Q which is removed after the reaction, with halobenzene derivatives of the formula III, 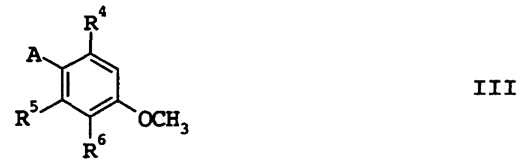 where the variables R⁴, R⁵ and R⁶ are as defined for formula I and A is halogen, and A is converted, in an intermediate step, into a group B, where B is a suitable metal or a suitable halometal group.

9. A process for preparing compounds of the formula I as claimed in claim 1 in which X is oxygen, by reduction of benzophenone derivatives of the formula IV, 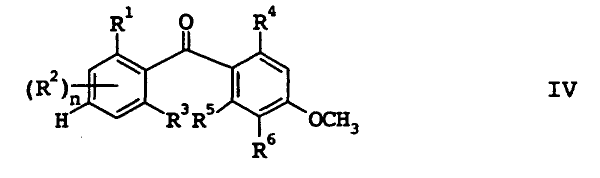 where the variables are as defined for formula I or denote a group which, after the reaction, can be converted into these radicals using hydrides or by catalytic reduction with H₂.

10. A composition suitable for controlling phytopathogenic harmful fungi, which comprises a solid or liquid carrier and a compound of the formula I as claimed in claim 1.

11. The use of the compounds I as claimed in claim 1 for preparing a composition suitable for controlling phytopathogenic harmful fungi.

12. A method for controlling phytopathogenic fungi, which comprises treating the fungi or the materials, plants, the soil or the seed to be protected against fungal attack with an effective amount of a compound of the formula I as claimed in claim 1.

## Revendications

1. Dérivés de benzhydryle de formule I, 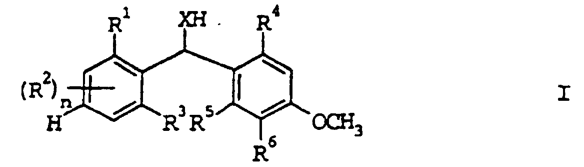 dans laquelle l'indice et les variables prennent la signification suivante :
X représente un atome d'oxygène ou de soufre;
R¹,R³ représentent un atome d'halogène, un groupe cyano, nitro, hydroxy, mercapto, amino, alkyle en C₁ à C₆, alcényle en C₂ à C₆, alcynyle en C₂ à C₆, alcoxy en C₁ à C₆, alcényloxy en C₂ à C₆, alcynyloxy en C₂ à C₆, (alkyle en C₁ à C₆)carbonyloxy, formyloxy, alkylthio en C₁ à C₆, alcénylthio en C₂ à C₆, alcynylthio en C₂ à C₆, alkylamino en C₁ à C₆, di(alkyle en C₁ à C₆)amino, (alkyle en C₁ à C₆)carbonyle ou formyle, les atomes de carbone dans les résidus cités pouvant être partiellement ou totalement halogénés;
R² représente un atome d'halogène, un groupe cyano, nitro, hydroxy, mercapto, amino, alkyle en C₁ à C₆, alcoxy en C₁ à C₆, halogénoalkyle en Ci à C₆ ou halogénoalcoxy en C₁ à C₆, les groupes R² pouvant être différents, lorsque n=2;
R⁴ représente un groupe alkyle en C₁ à C₆, alcényle en C₂ à C₆ ou alcynyle en C₂ à C₆, les atomes de carbone dans ces résidus pouvant être non substitués ou bien partiellement ou totalement halogénés;
R⁵,R⁶ représentent un groupe hydroxy, alkyle en C₁ à C₆, alcényle en C₂ à C₆, halogénoalkyle en C₁ à C₆, halogénoalcényle en C₂ à C₆, alcoxy en C₁ à C₆, alcényloxy en C₂ à C₆, halogénoalcoxy en C₁ à C₆, halogénoalcényloxy en C₂ à C₆, cycloalkyle en C₃ à C₈, (cycloalkyle en C₃ à C₈)-(alkyle en C₁ à C₃), cycloalcoxy en C₃ à C₈ ou (cycloalkyle en C₃ à C₈)-(alcoxy en C₁ à C₃);
n vaut 0, 1 ou 2.

2. Composés de formule 1 selon la revendication 1, dans laquelle X représente un atome d'oxygène.

3. Composés de formule 1 selon les revendications 1 ou 2, dans laquelle les variables prennent la signification suivante :
R¹,R³ représentent indépendamment l'un de l'autre, un atome d'halogène, un groupe hydroxy, amino, mercapto, nitro, cyano, alkyle en C₁ à C₆, halogénoalkyle en C₁ à C₆, alcoxy en C₁ à C₆, halogénoalcoxy en C₁ à C₆, (alkyle en C₁ à C₆)carbonyloxy, (halogénoalkyle en C₁ à C₆)carbonyloxy, alkylthio en C₁ à C₆, halogénoalkylthio en C₁ à C₆, (alkyle en C₁ à C₆)carbonyle ou (halogénoalkyle en C₁ à C₆)carbonyle.

4. Composés de formule 1 selon les revendications 1 à 3, dans laquelle
R² représente un atome d'halogène, un groupe hydroxy, alkyle en C₁ à C₆, halogénoalkyle en C₁ à C₆, alcoxy en C₁ à C₆ ou halogénoalcoxy en C₁ à C₆.

5. Composés de formule 1 selon les revendications 1 à 4, dans laquelle
R⁴ représente un groupe alkyle en C₁ à C₆ ou halogénoalkyle en C₁ à C₆.

6. Composés de formule 1 selon les revendications 1 à 5, dans laquelle
R⁵,R⁶ représentent un groupe hydroxy, alcoxy en C₁ à C₆, alcényloxy en C₂ à C₆, halogénoalcoxy en C₁ à C₆ ou halogénoalcényloxy en C₂ à C₆.

7. Composés de formule 1 selon les revendications 1 ou 2, dans laquelle les variables prennent la signification suivante :
R¹,R³ représentent un atome d'halogène, un groupe hydroxy, amino, mercapto, alkyle en C₁ à C₆, halogénoalkyle en C₁ à C₆, alcoxy en C₁ à C₆, halogénoalcoxy en C₁ à C₆ ou (alkyle en C₁ à C₆)carbonyle;
R² représente un atome d'halogène, un groupe hydroxy, alkyle en C₁ à C₆, halogénoalkyle en C₁ à C₆, alcoxy en C₁ à C₆ ou halogénoalcoxy en C₁ à C₆;
R⁴ représente un groupe méthyle;
R⁵,R⁶ représentent un groupe alcoxy en C₁ à C₆;
n vaut 0 ou 1.

8. Procédé de préparation des composés de formule 1 selon la revendication 1, dans laquelle X représente un atome d'oxygène, par conversion d'aldéhydes de formule II, 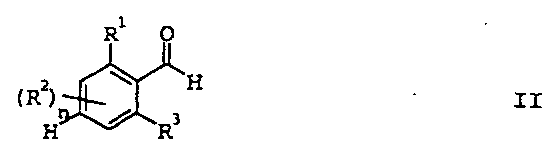 dans laquelle les variables R¹, R², R³ et n prennent la signification indiquée et qui peuvent être protégés par un groupe protecteur Q à cliver après la conversion, avec des dérivés halogénobenzène de formule III 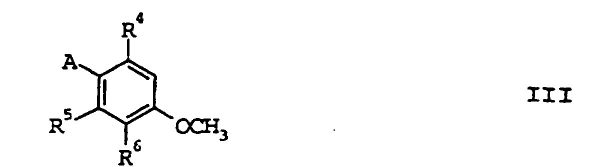 dans laquelle les variables R⁴, R⁵, R⁶ prennent la signification indiquée pour la formule 1 et A représente un atome d'halogène, et A est transformé dans une étape intermédiaire en un groupe B, B représentant un métal approprié ou un groupe halogénométallique approprié.

9. Procédé de préparation des composés de formule 1 selon la revendication 1, dans laquelle X représente un atome d'oxygène, par réduction des dérivés de benzophénone de formule IV, 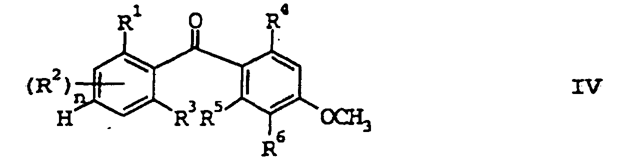 dans laquelle les variables prennent la signification indiquée pour la formule I ou représentent un groupe qui peut être transformé en outre après la réaction, par des hydrures ou à l'aide d'une réduction catalytique par H₂.

10. Agent convenant à lutte contre les champignons nuisibles phytopathogènes, contenant un véhicule solide ou liquide et un composé de formule générale I selon la revendication 1.

11. Utilisation des composés I selon la revendication 1, pour préparer un agent convenant à lutte contre les champignons nuisibles phytopathogènes.

12. Procédé de lutte contre les champignons nuisibles phytopathogènes, **caractérisé en ce que** l'on traite les champignons ou bien les matériaux, les plantes, le sol ou les semences à protéger contre une infection par des champignons, par une quantité efficace d'un composé de formule générale I selon la revendication 1.
